(19) Europäisches Patentamt
European Patent Office
Office européen des brevets

(11) Veröffentlichungsnummer: **0 493 671 B1**

# (12) EUROPÄISCHE PATENTSCHRIFT

(45) Veröffentlichungstag der Patentschrift: **01.03.95**

(51) Int. Cl.$^6$: **A01N 43/40**

(21) Anmeldenummer: **91119903.2**

(22) Anmeldetag: **22.11.91**

## (54) Verwendung von Pyridinium-Salzen zum Schutz von Planzen gegen Befall durch Mikroorganismen.

(30) Priorität: **05.12.90 DE 4038741**

(43) Veröffentlichungstag der Anmeldung:
**08.07.92 Patentblatt 92/28**

(45) Bekanntmachung des Hinweises auf die Patenterteilung:
**01.03.95 Patentblatt 95/09**

(84) Benannte Vertragsstaaten:
**BE CH DE ES FR GB IT LI NL**

(56) Entgegenhaltungen:
**EP-A- 0 155 574**
**EP-A- 0 268 775**
**US-A- 3 285 926**

**CHEMICAL ABSTRACTS, Band 103, Nr. 5, 5. August 1985, Seite 152, Zusammenfassung Nr. 33399k, Columbus, Ohio, US; E.P. NESYNOV et al.: "Fungicidal activity of quaternary salts of beta-dialkylaminoethyl esters of nicotinic acid", &FIZIOL. AKT. VESHCHESTVA 1984, 16, 66-7**

(73) Patentinhaber: **BAYER AG**
**D-51368 Leverkusen (DE)**

(72) Erfinder: **Lunkenheimer, Winfried, Dr.**
**Funckstrasse 49**
**W-5600 Wuppertal (DE)**
Erfinder: **Wollweber, Detlef, Dr.**
**Paul-Ehrlich-Strasse 17**
**W-5600 Wuppertal (DE)**
Erfinder: **Dehne, Heinz-Wilhelm, Dr.**
**Krischer Strasse 81**
**W-4019 Monheim (DE)**
Erfinder: **Dutzmann, Stefan, Dr.**
**Kosenberg 10**
**W-4010 Hiden (DE)**
Erfinder: **Hänssler, Gerd, Dr.**
**Am Arenzberg 58a**
**W-5090 Leverkusen 3 (DE)**
Erfinder: **Mauler-Machnik, Astrid, Dr.**
**Neuenkamper Weg 46a**
**W-5653 Leichlingen (DE)**
Erfinder: **Schulz, Uta, Dr.**
**Metzholz 38**
**W-5643 Leichlingen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

CENTRAL PATENTS INDEX, BASIC ABSTRACTS JOURNAL, AGDOC, Woche 33, 1971,Accession Nr. 71-54316S, Derwent Publications Ltd, London, GB;& JP-A-7 128 800 (INAGAMI) 20-08-1971

## Beschreibung

Die vorliegende Erfindung betrifft die Verwendung von teilweise bekannten Pyridinium-Salzen zur Erzeugung von Resistenz in Pflanzen gegen Befall durch unerwünschte Mikroorganismen.

Es ist bereits bekannt, daß sich bestimmte Isonicotincarbonsäure-Derivate zur Erzeugung von Resistenz in Pflanzen gegen Befall durch phytopathogene Mikroorganismen einsetzen lassen (vgl. EP-A 0 268 775). So kann zum Beispiel 2,6-Dichlor-isonicotinsäuremethylester für den genannten Zweck verwendet werden. Die Wirkung dieses Stoffes ist jedoch nicht immer befriedigend.

Aus dem Artikel, über den in Chem. Abstr. 103, 33 399 k (1985) berichtet wird, geht hervor, daß quarternäre Salze von $\beta$-Dialkylaminoethylnicotinsäureestern als Fungizide eingesetzt werden können. Im Alkylteil unsubstituierte Ester oder Aralkylester werden aber nicht beschrieben. Außerdem finden sich keine Hinweise auf eine resistenzinduzierende Wirkung der betreffenden Stoffe.

Aus der EP-A 0 155 574 sind bestimmte Pyridinium-Salze mit fungiziden und bakteriziden Eigenschaften bekannt. Es werden aber keine Verbindungen offenbart, in denen der mit dem Stickstoff des Pyridin-Ringes verbundene Rest für Alkyl oder gegebenenfalls substituiertes Aralkyl steht.

Der US-A 3 285 926 läßt sich entnehmen, daß Perfluordioxocycloalkyl-Derivate sich im Materialschutz einsetzen lassen. Bei den Stoffen handelt es sich um Betaine, die sich vom Pyridin ableiten. Herkömmliche Salze des Pyridins werden aber nicht erwähnt.

Schließlich wird in der JP-A 71-28 800 mitgeteilt, daß bestimmte Salze von Nicotinsäureestern zur Konservierung und zur Desinfektion geeignet sind. Eine resistenzinduzierende Aktivität dieser Stoffe wird aber nicht offenbart.

Es wurde nun gefunden, daß die teilweise bekannten Pyridinium-Salze der Formel

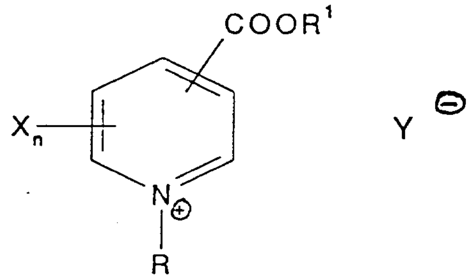

(I)

in welcher

- R für Alkyl mit 1 bis 8 Kohlenstoffatomen oder für Aralkyl mit 6 bis 10 Kohlenstoffatomen im Arylteil und 1 bis 4 Kohlenstoffatomen im Alkylteil steht,
- $R^1$ für Alkyl mit 1 bis 8 Kohlenstoffatomen oder für Aralkyl mit 6 bis 10 Kohlenstoffatomen im Arylteil und 1 bis 4 Kohlenstoffatomen im Alkylteil steht, wobei jeder dieser Reste im Arylteil einfach bis dreifach, gleichartig oder verschieden durch Halogen substituiert sein kann,
- X für Fluor, Chlor, Brom, Iod, Alkyl mit 1 bis 8 Kohlenstoffatomen, Halogenalkyl mit 1 bis 4 Kohlenstoffatomen und 1 bis 5 gleichen oder verschiedenen Halogenatomen, Alkyoxy mit 1 bis 8 Kohlenstoffatomen, Halogenalkoxy mit 1 bis 4 Kohlenstoffatomen und 1 bis 5 gleichen oder verschiedenen Halogenatomen, Hydroxy, Mercapto, Alkylthio mit 1 bis 8 Kohlenstoffatomen, Halogenalkylthio mit 1 bis 4 Kohlenstoffatomen und 1 bis 5 gleichen oder verschiedenen Halogenatomen, Amino, Alkylamino mit 1 bis 6 Kohlenstoffatomen, Dialkylamino mit 1 bis 6 Kohlenstoffatomen in jeder Alkylgruppe, Phenylamino, Benzylamino oder gegebenenfalls durch Halogen und/oder Alkyl mit 1 bis 4 Kohlenstoffatomen substituiertes Phenyl steht,
- n für 0, 1, 2, 3 oder 4 steht und
- $Y^\ominus$ für Halogenid, Alkylsulfat mit 1 bis 4 Kohlenstoffatomen, Tetrafluoroborat, für ein Äquivalent eines Sulfatanions, für Alkylsulfonat mit 1 bis 4 Kohlenstoffatomen, gegebenenfalls durch Methyl substituiertes Phenylsulfonat, Nitrat, Alkylcarboxylat mit 1 bis 4 Kohlenstoffatomen in der Alkylgruppe oder ein Äquivalent eines Oxalatanions steht,

sehr gut zur Erzeugung von Resistenz in Pflanzen gegen Befall durch unerwünschte Mikroorganismen verwendbar sind.

Überraschenderweise eignen sich die erfindungsgemäßen Pyridinium-Salze der Formel (I) besser zur Erzeugung von Resistenz in Pflanzen gegen Befall durch phytopathogene Mikroorganismen als die konstitutionell ähnlichsten Verbindungen, die aus dem Stand der Technik bekannt und für den gleichen

Zweck einsetzbar sind. So übertreffen die erfindungsgemäßen Stoffe zum Beispiel den 2,6-Dichlor-isonicotinsäuremethylester bezüglich der resistenzerzeugenden Wirkung in Pflanzen gegen Befall durch unerwünschte Mikroorganismen.

Die erfindungsgemäß verwendbaren Pyridinium-Salze sind durch die Formel (I) allgemein definiert.

Wenn n für 2, 3 oder 4 steht, können die durch X bezeichneten Substituenten gleichartig oder verschieden sein.

R steht bevorzugt für Alkyl mit 1 bis 6 Kohlenstoffatomen oder für Phenylalkyl oder Naphthylalkyl mit jeweils 1 oder 2 Kohlenstoffatomen im Alkylteil.

$R^1$ steht bevorzugt für Alkyl mit 1 bis 6 Kohlenstoffatomen oder für Phenylalkyl mit 1 oder 2 Kohlenstoffatomen im Alkylteil, wobei jeder dieser Reste im Phenylteil einfach bis dreifach, gleichartig oder verschieden substituiert sein kann durch Fluor, Chlor, Brom .

X steht bevorzugt für Fluor, Chlor, Brom, Iod, Alkyl mit 1 bis 6 Kohlenstoffatomen, Halogenalkyl mit 1 oder 2 Kohlenstoffatomen und 1 bis 3 gleichen oder verschiedenen Fluor-, Chlor-und/oder Bromatomen, Alkoxy mit 1 bis 6 Kohlenstoffatomen, Halogenalkoxy mit 1 oder 2 Kohlenstoffatomen und 1 bis 3 gleichen oder verschiedenen Fluor-, Chlor- und/oder Bromatomen, Hydroxy, Mercapto, Alkylthio mit 1 bis 6 Kohlenstoffatomen, Halogenalkylthio mit 1 oder 2 Kohlenstoffatomen und 1 bis 3 gleichen oder verschiedenen Fluor-, Chlor- und/oder Bromatomen, Amino, Alkylamino mit 1 bis 4 Kohlenstoffatomen, Dialkylamino mit 1 bis 4 Kohlenstoffatomen in jeder Alkylgruppe, Phenylamino, Benzylamino oder für gegebenenfalls einfach oder zweifach, gleichartig oder verschieden durch Fluor, Chlor, Methyl und/oder Ethyl substituiertes Phenyl.

n steht für 0, 1, 2, 3 oder 4.

$Y^{\ominus}$ steht bevorzugt für Chlorid, Bromid, Iodid, Methylsulfat, Ethylsulfat, Tetrafluoroborat, für ein Äquivalent eines Sulfatanions für Methylsulfonat, Ethylsulfonat, Phenylsulfonat, 4-Methylphenylsulfonat, Nitrat, Acetat oder ein Äquivalent eines Oxalat-Anions.

Als Beispiele für erfindungsgemäß verwendbare Pyridinium-Salze seien die in den folgenden Tabellen aufgeführten Stoffe genannt.

Tabelle 1

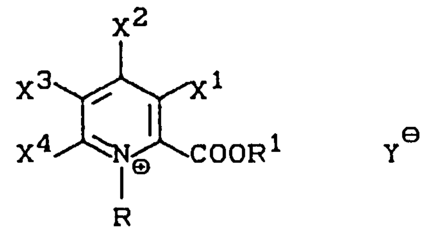

(Ia)

| R | $X^1$ | $X^2$ | $X^3$ | $X^4$ | $R^1$ | Y |
|---|---|---|---|---|---|---|
| $-C_3H_7-n$ | H | H | H | H | $CH_3$ | I |
| $-C_4H_9-n$ | " | " | " | " | " | Br |
| $-C_5H_{11}-n$ | " | " | " | " | " | Br |
| $-C_6H_{13}-n$ | " | " | " | " | " | Cl |
| $-C_3H_7-iso$ | " | " | " | " | " | Cl |
| $-C_3H_7-n$ | " | " | " | " | $C_2H_5$ | Br |
| $-C_4H_9-n$ | H | H | H | H | $C_2H_5$ | Cl |
| $-C_5H_{11}-n$ | " | " | " | " | " | Br |
| $-C_6H_{13}-n$ | " | " | " | " | " | Br |
| $-C_3H_7-iso$ | " | " | " | " | " | Br |

<u>Tabelle 1</u> (Fortsetzung)

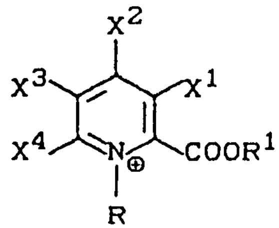 $Y^{\ominus}$ (Ia)

| R | $X^1$ | $X^2$ | $X^3$ | $X^4$ | $R^1$ | Y |
|---|---|---|---|---|---|---|
| $-C_2H_5$ | H | H | H | H | $-C_2H_5$ | J |
| $-C_2H_5$ | " | " | " | " | $-C_3H_7$-n | J |
| $-C_2H_5$ | " | " | " | " | $-C_3H_7$-iso | |
| $-C_2H_5$ | " | " | " | " | $-C_4H_9$-n | J |
| $-C_2H_5$ | " | " | " | " | $-C_4H_9$-sek. | J |
| $-C_2H_5$ | H | H | H | H | $-C_4H_9$-iso | J |
| $-C_2H_5$ | " | " | " | " | $-C_4H_9$-tert | J |

<u>Tabelle 1</u> (Fortsetzung)

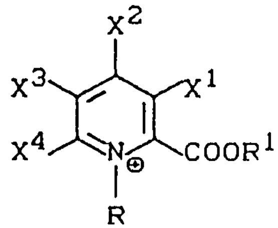

$Y^{\ominus}$

(Ia)

| R | $X^1$ | $X^2$ | $X^3$ | $X^4$ | $R^1$ | Y |
|---|---|---|---|---|---|---|
| $-C_2H_5$ | H | H | H | H | 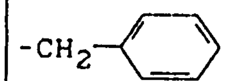 | J |
| $-C_2H_5$ | " | " | " | " | 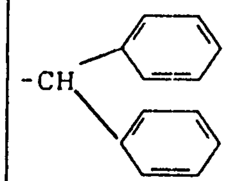 | J |
| $-C_2H_5$ | H | H | H | H | 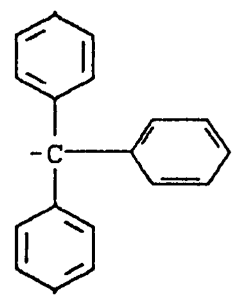 | J |

Tabelle 1 (Fortsetzung)

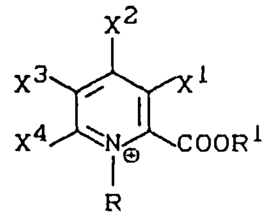

$Y^{\ominus}$

(Ia)

| R | $X^1$ | $X^2$ | $X^3$ | $X^4$ | $R^1$ | Y |
|---|---|---|---|---|---|---|
| $-C_2H_5$ | H | H | H | H | $-CH_3$ | $C_2H_5OSO_3^-$ |
| $-C_2H_5$ | " | " | " | " | $-CH_3$ | 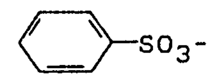 |
| $-C_2H_5$ | H | H | H | H | $-CH_3$ | $CH_3-COO^-$ |
| $-C_2H_5$ | " | " | " | " | $-CH_3$ | 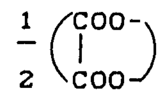 |
| $-C_2H_5$ | " | " | " | " | $-CH_3$ | $\frac{1}{2}$ $SO_4$ |
| $-C_2H_5$ | " | " | " | " | $-CH_3$ | $HSO_4^-$ |
| $-C_2H_5$ | " | " | " | " | $-CH_3$ | $NO_3$ |
| $-C_2H_5$ | " | " | " | " | $-CH_3$ | $BF_4$ |

<u>Tabelle 1</u> (Fortsetzung)

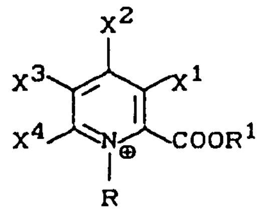

$Y^{\ominus}$

(Ia)

| R | $X^1$ | $X^2$ | $X^3$ | $X^4$ | $R^1$ | Y |
|---|---|---|---|---|---|---|
| $-CH_3$ | $CH_3$ | " | " | " | $-CH_3$ | J |
| $-CH_3$ | H | $CH_3$ | H | H | $-C_2H_5$ | J |
| $-CH_3$ | H | H | $CH_3$ | H | $-CH_3$ | $CH_3OSO_3$ |
| $-CH_3$ | H | H | H | $CH_3$ | $-C_2H_5$ | $CH_3OSO_3$ |
| $-CH_3$ | $CH_3$ | $CH_3$ | H | H | $-C_2H_5$ | $CH_3OSO_3$ |
| $-CH_3$ | $CH_3$ | H | $CH_3$ | H | $-CH_3$ | J |
| $-CH_3$ | $CH_3$ | H | H | $CH_3$ | $-CH_3$ | J |
| $-CH_3$ | H | $CH_3$ | H | H | $-CH_3$ | J |

**Tabelle 1** (Fortsetzung)

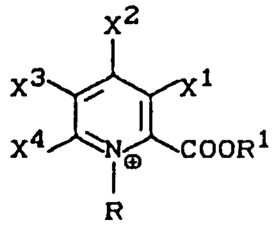 $Y^{\ominus}$ (Ia)

| R | $X^1$ | $X^2$ | $X^3$ | $X^4$ | $R^1$ | Y |
|---|---|---|---|---|---|---|
| $-CH_3$ | H | $CH_3$ | H | $CH_3$ | $-CH_3$ | J |
| $-CH_3$ | H | H | $CH_3$ | $CH_3$ | $-CH_3$ | J |
| $-CH_3$ | $CH_3$ | $CH_3$ | H | $CH_3$ | $-C(CH_3)_3$ | J |
| $-CH_3$ | $CH_3$ | H | H | H | $-C_2H_5$ | J |
| $-CH_3$ | H | $CH_3$ | H | H | $-CH_3$ | J |
| $-CH_3$ | $CH_3$ | H | $CH_3$ | H | $-C_2H_5$ | J |
| $-CH_3$ | $CH_3$ | H | H | $CH_3$ | $-C_2H_5$ | J |
| $-CH_3$ | H | $CH_3$ | $CH_3$ | H | $-C(CH_3)_3$ | J |
| $-CH_3$ | $CH_3$ | $CH_3$ | H | $CH_3$ | $-CH_3$ | $CH_3OSO_3$ |

Tabelle 1 (Fortsetzung)

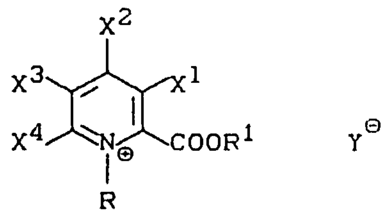

(Ia)

| R | $X^1$ | $X^2$ | $X^3$ | $X^4$ | $R^1$ | Y |
|---|---|---|---|---|---|---|
| $-CH_3$ | $CH_3$ | $CH_3$ | $CH_3$ | $CH_3$ | $-CH_3$ | $CH_3OSO_3$ |
| $-CH_3$ | $C_2H_5$ | H | H | H | $-CH_3$ | J |
| $-CH_3$ | H | H | H | $C_2H_5$ | $-CH_3$ | J |
| $-CH_3$ | H | H | H | $C_3H_7$-n | $-CH_3$ | J |
| $-CH_3$ | Cl | H | H | H | $-CH_3$ | J |
| $-CH_3$ | H | Cl | H | H | $-CH_3$ | J |
| $-CH_3$ | H | H | Cl | H | $-CH_3$ | J |
| $-CH_3$ | H | H | H | Cl | $-CH_3$ | J |
| $-CH_3$ | Cl | Cl | H | H | $-CH_3$ | J |
| $-CH_3$ | Cl | H | Cl | H | $-CH_3$ | J |

<u>Tabelle 1</u> (Fortsetzung)

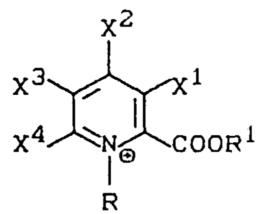

$Y^{\ominus}$

(Ia)

| R | $X^1$ | $X^2$ | $X^3$ | $X^4$ | $R^1$ | Y |
|---|---|---|---|---|---|---|
| $-CH_3$ | H | Cl | H | Cl | $-CH_3$ | J |
| $-CH_3$ | H | H | Cl | Cl | $-CH_3$ | J |
| $-CH_3$ | Br | H | H | H | $-CH_3$ | J |
| $-CH_3$ | H | H | Br | H | $-CH_3$ | J |
| $-CH_3$ | F | H | H | H | $-CH_3$ | J |
| $-CH_3$ | H | H | H | F | $-CH_3$ | J |
| $-CH_3$ | H | H | F | H | $-CH_3$ | J |
| $-CH_3$ | $CH_3$ | $CH_3$ | Cl | $CH_3$ | $-CH_3$ | J |
| $-CH_3$ | $CH_3$ | $CF_3$ | H | $CF_3$ | $-CH_3$ | J |
| $-CH_3$ | H | H | H | $CF_3$ | $-CH_3$ | J |

Tabelle 1 (Fortsetzung)

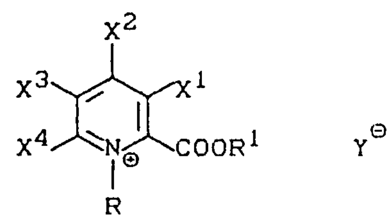

(Ia)

| R | $X^1$ | $X^2$ | $X^3$ | $X^4$ | $R^1$ | Y |
|---|---|---|---|---|---|---|
| $-CH_3$ | $CF_3$ | $CH_3$ | $CH_3$ | $CH_3$ | $-CH_3$ | J |
| $-CH_3$ | $CH_3$ | $C_2F_5$ | H | $C_2F_5$ | $-CH_3$ | J |
| $-CH_3$ | $OCF_3$ | $CH_3$ | H | $CH_3$ | $-CH_3$ | J |
| $-CH_3$ | $CH_3$ | $OCF_3$ | H | $OCF_3$ | $-CH_3$ | J |
| $-CH_3$ | $SCF_3$ | H | H | H | $-CH_3$ | J |
| $-CH_3$ | H | H | OH | H | $-CH_3$ | J |
| $-CH_3$ | H | OH | H | H | $-CH_3$ | J |
| $-CH_3$ | OH | H | H | H | $-CH_3$ | J |
| $-CH_3$ | H | SH | H | H | $-CH_3$ | J |

<u>Tabelle 1</u> (Fortsetzung)

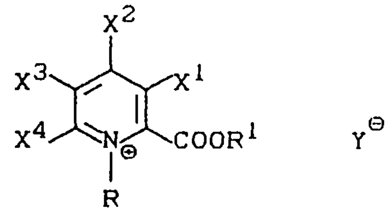

(Ia)

| R | $X^1$ | $X^2$ | $X^3$ | $X^4$ | $R^1$ | Y |
|---|---|---|---|---|---|---|
| $-CH_3$ | H | H | SH | H | $-CH_3$ | J |
| $-CH_3$ | SH | H | H | H | $-CH_3$ | J |
| $-CH_3$ | $NH_2$ | H | H | H | $-CH_3$ | J |
| $-CH_3$ | H | H | $NH_2$ | H | $-CH_3$ | J |
| $-CH_3$ | H | H | $OCH_3$ | H | $-CH_3$ | J |
| $-CH_3$ | H | $OCH_3$ | H | H | $-CH_3$ | J |
| $-CH_3$ | $OCH_3$ | H | H | H | $-CH_3$ | J |
| $-CH_3$ | $OC_2H_5$ | H | H | H | $-CH_3$ | J |
| $-CH_3$ | H | $SCH_3$ | H | H | $-CH_3$ | J |
| $-CH_3$ | H | H | $SCH_3$ | H | $-CH_3$ | J |

Tabelle 1 (Fortsetzung)

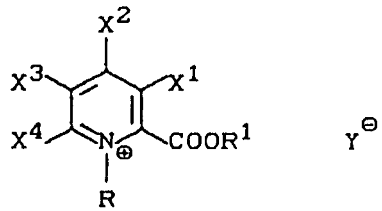

(Ia)

| R | $X^1$ | $X^2$ | $X^3$ | $X^4$ | $R^1$ | Y |
|---|---|---|---|---|---|---|
| $-CH_3$ | $SC_2H_5$ | H | H | H | $-CH_3$ | J |
| $-CH_3$ | $NH-CH_3$ | H | H | H | $-CH_3$ | J |
| $-CH_3$ | $N(CH_3)_2$ | H | H | H | $-CH_3$ | J |
| $-CH_3$ | H | H | $N(C_2H_5)_2$ | H | $-CH_3$ | J |
| $-CH_3$ | H | H | H | 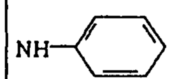 | $-CH_3$ | J |
| $-CH_3$ | 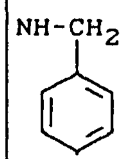 | H | H | H | $-CH_3$ | J |
| $-CH_3$ | H | 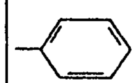 | H | 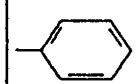 | $-CH_3$ | J |
| $-CH_3$ | 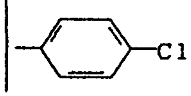 | $CH_3$ | H | $CH_3$ | $-CH_3$ | J |

Tabelle 1 (Fortsetzung)

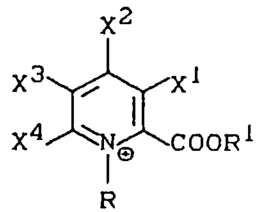 $Y^{\ominus}$ (Ia)

| R | $X^1$ | $X^2$ | $X^3$ | $X^4$ | $R^1$ | Y |
|---|---|---|---|---|---|---|
| $-CH_3$ | 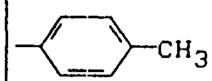 | H | H | 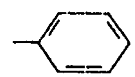 | $-CH_3$ | J |
| $-CH_3$ | 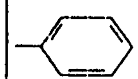 | $CH_3$ | H | 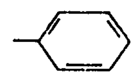 | $-CH_3$ | J |
| $-C_2H_5$ | $CH_3$ | H | H | H | $-CH_3$ | J |
| $-C_2H_5$ | H | $CH_3$ | H | H | $-C_2H_5$ | J |
| $-C_2H5$ | H | H | $CH_3$ | H | $-CH_3$ | $C_2H_5OSO_3$ |
| $-C_2H_5$ | H | H | H | $CH_3$ | $-C_2H_5$ | $C_2H_5OSO_3$ |
| $-C_2H_5$ | $CH_3$ | $CH_3$ | H | H | $-C_2H_5$ | $C_2H_5OSO_3$ |
| $-C_2H_5$ | $CH_3$ | H | $CH_3$ | H | $-CH_3$ | J |
| $-C_2H_5$ | $CH_3$ | H | H | $CH_3$ | $-CH_3$ | J |

<u>Tabelle 1</u> (Fortsetzung)

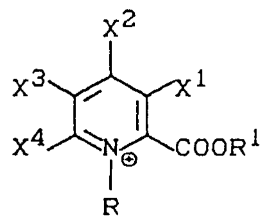 $Y^{\ominus}$ (Ia)

| R | $X^1$ | $X^2$ | $X^3$ | $X^4$ | $R^1$ | Y |
|---|---|---|---|---|---|---|
| $-C_2H_5$ | H | $CH_3$ | $CH_3$ | H | $-CH_3$ | J |
| $-C_2H_5$ | H | $CH_3$ | H | $CH_3$ | $-CH_3$ | J |
| $-C_2H_5$ | H | H | $CH_3$ | $CH_3$ | $-CH_3$ | J |
| $-C_2H_5$ | $CH_3$ | $CH_3$ | H | $CH_3$ | $-C(CH_3)_3$ | J |
| $-C_2H_5$ | Cl | H | H | H | $-CH_3$ | J |

Tabelle 1 (Fortsetzung)

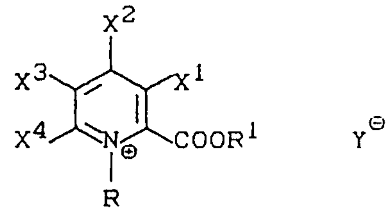

(Ia)

| R | $X^1$ | $X^2$ | $X^3$ | $X^4$ | $R^1$ | Y |
|---|---|---|---|---|---|---|
| $-C_2H_5$ | H | Cl | H | H | $-CH_3$ | J |
| $-C_2H_5$ | H | H | Cl | H | $-CH_3$ | J |
| $-C_2H_5$ | H | H | H | Cl | $-CH_3$ | J |
| $-C_2H_5$ | Cl | Cl | H | H | $-CH_3$ | J |
| $-C_2H_5$ | Cl | H | Cl | H | $-CH_3$ | J |
| $-C_2H_5$ | H | Cl | H | Cl | $-CH_3$ | J |
| $-C_2H_5$ | H | H | Cl | Cl | $-CH_3$ | J |
| $-C_2H_5$ | Br | H | H | H | $-CH_3$ | J |
| $-C_2H_5$ | H | H | Br | H | $-CH_3$ | J |
| $-C_2H_5$ | F | H | H | H | $-CH_3$ | J |

<u>Tabelle 1</u> (Fortsetzung)

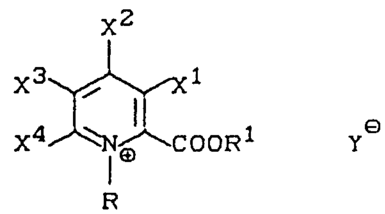

(Ia)

| R | $X^1$ | $X^2$ | $X^3$ | $X^4$ | $R^1$ | Y |
|---|---|---|---|---|---|---|
| $-C_2H_5$ | H | H | H | F | $-CH_3$ | J |
| $-C_2H_5$ | H | H | F | H | $-CH_3$ | J |
| $-C_2H_5$ | $CH_3$ | $CH_3$ | Cl | $CH_3$ | $-CH_3$ | J |
| $-C_2H_5$ | $CH_3$ | $CF_3$ | H | $CF_3$ | $-CH_3$ | J |
| $-C_2H_5$ | H | H | H | $CF_3$ | $-CH_3$ | J |
| $-C_2H_5$ | $CF_3$ | $CH_3$ | $CH_3$ | $CH_3$ | $-CH_3$ | J |
| $-C_2H_5$ | $CH_3$ | $C_2F_5$ | H | $C_2F_5$ | $-CH_3$ | J |
| $-C_2H_5$ | $OCF_3$ | $CH_3$ | H | $CH_3$ | $-CH_3$ | J |
| $-C_2H_5$ | $CH_3$ | $OCF_3$ | H | $OCF_3$ | $-CH_3$ | J |
| $-C_2H_5$ | $SCF_3$ | H | H | H | $-CH_3$ | J |

Tabelle 1 (Fortsetzung)

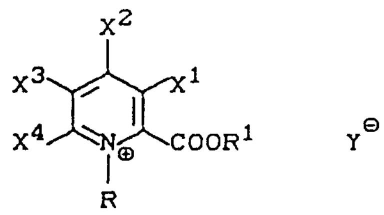

(Ia)

| R | $X^1$ | $X^2$ | $X^3$ | $X^4$ | $R^1$ | Y |
|---|---|---|---|---|---|---|
| $-C_2H_5$ | H | H | OH | H | $-CH_3$ | J |
| $-C_2H_5$ | H | OH | H | H | $-CH_3$ | J |
| $-C_2H_5$ | OH | H | H | H | $-CH_3$ | J |
| $-C_2H_5$ | H | SH | H | H | $-CH_3$ | J |
| $-C_2H_5$ | H | H | SH | H | $-CH_3$ | J |
| $-C_2H_5$ | SH | H | H | H | $-CH_3$ | J |
| $-C_2H_5$ | $NH_2$ | H | H | H | $-CH_3$ | J |
| $-C_2H_5$ | H | H | $NH_2$ | H | $-CH_3$ | J |
| $-C_2H_5$ | H | H | $OCH_3$ | H | $-CH_3$ | J |
| $-C_2H_5$ | H | $OCH_3$ | H | H | $-CH_3$ | J |

Tabelle 1 (Fortsetzung)

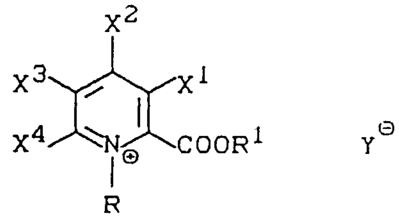

(Ia)

| R | $X^1$ | $X^2$ | $X^3$ | $X^4$ | $R^1$ | Y |
|---|---|---|---|---|---|---|
| $-C_2H_5$ | $OCH_3$ | H | H | H | $-CH_3$ | |
| $-C_2H_5$ | $OC_2H_5$ | H | H | H | $-CH_3$ | J |
| $-C_2H_5$ | H | $SCH_3$ | H | H | $-CH_3$ | J |
| $-C_2H_5$ | H | H | $SCH_3$ | H | $-CH_3$ | J |
| $-C_2H_5$ | $SC_2H_5$ | H | H | H | $-CH_3$ | J |
| $-C_2H_5$ | $NH-CH_3$ | H | H | H | $-CH_3$ | J |
| $-C_2H_5$ | $N(CH_3)_2$ | H | H | H | $-CH_3$ | J |
| $-C_2H_5$ | H | H | $N(C_2H_5)_2$ | H | $-CH_3$ | J |
| $-C_2H_5$ | H | H | H | NH- 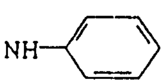 | $-CH_3$ | J |

Tabelle 1 (Fortsetzung)

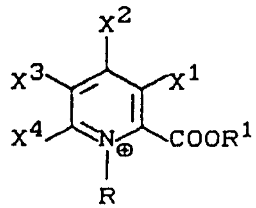

$Y^{\ominus}$

(Ia)

| R | $X^1$ | $X^2$ | $X^3$ | $X^4$ | $R^1$ | Y |
|---|---|---|---|---|---|---|
| $-C_2H_5$ | $-NH-CH_2$ 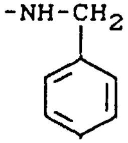 | H | H | H | $-CH_3$ | J |
| $-C_2H_5$ | H | 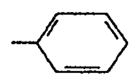 | H | 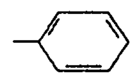 | $-CH_3$ | J |
| $-C_2H_5$ | 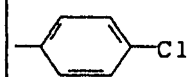 | $CH_3$ | H | $CH_3$ | $-CH_3$ | J |
| $-C_2H_5$ | 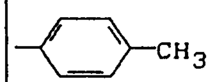 | H | H | 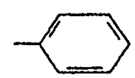 | $-CH_3$ | J |
| $-C_2H_5$ | 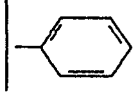 | $CH_3$ | H | 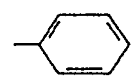 | $-CH_3$ | J |

Tabelle 1 (Fortsetzung)

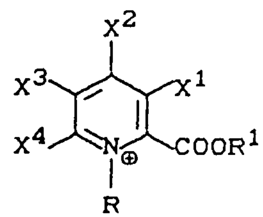

$Y^{\ominus}$ (Ia)

| R | $X^1$ | $X^2$ | $X^3$ | $X^4$ | $R^1$ | Y |
|---|---|---|---|---|---|---|
| $-C_3H_7$-n | $CH_3$ | H | H | H | $-CH_3$ | J |
| $-C_4H_9$-n | H | $CH_3$ | H | H | $-C_2H_5$ | Br |
| $-C_3H_7$-n | H | Cl | H | Cl | $-C_3H_7$-n | Br |
| $-C_6H_{13}$-n | H | H | Cl | Cl | $-CH_3$ | Cl |
| $-CH_2-CH_2-$ 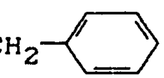 | H | H | H | F | $-CH_3$ | Cl |
| $-CH_2$ 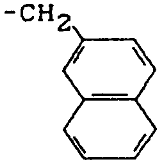 | $CF_3$ | $CH_3$ | $CH_3$ | $CH_3$ | $-CH_3$ | Cl |
| $-C(CH_3)_3$ | H | SH | H | H | $-CH_3$ | Cl |

<u>Tabelle 1</u> (Fortsetzung)

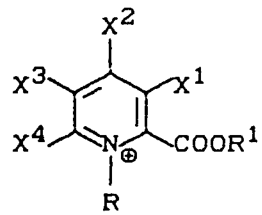

$Y^{\ominus}$ (Ia)

| R | $X^1$ | $X^2$ | $X^3$ | $X^4$ | $R^1$ | Y |
|---|---|---|---|---|---|---|
| $-C_4H_9$-n | H | H | $NH_2$ | H | $-C_2H_5$ | Br |
| $-C_5H_{11}$-n | $NH-CH_3$ | H | H | H | $-CH_3$ | Br |
| $-C_6H_{13}$-n | 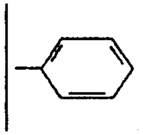 | $CH_3$ | H | 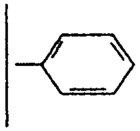 | $-CH_3$ | Cl |

Tabelle 2

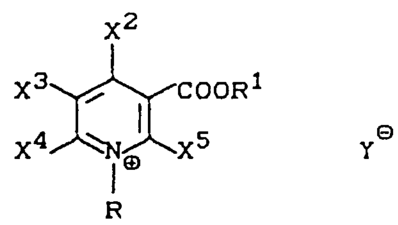

(Ib)

| R | $X^5$ | $X^2$ | $X^3$ | $X^4$ | $R^1$ | Y |
|---|---|---|---|---|---|---|
| $-C_3H_7$-n | H | H | H | H | $-CH_3$ | J |
| $-C_4H_9$-n | " | " | " | " | $-CH_3$ | Br |
| $-C_5H_{11}$-n | " | " | " | " | $-CH_3$ | Br |
| $-C_6H_{13}$-n | " | " | " | " | $-CH_3$ | Cl |
| $-C_3H_7$-iso | " | " | " | " | $-CH_3$ | Cl |
| $-C_4H_9$-iso | " | " | " | " | $-CH_3$ | Br |
| $-C_4H_9$-tert. | " | " | " | " | $-CH_3$ | Cl |

Tabelle 2 (Fortsetzung)

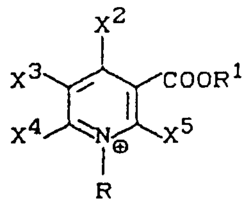

$Y^{\ominus}$ (Ib)

| R | $X^5$ | $X^2$ | $X^3$ | $X^4$ | $R^1$ | Y |
|---|---|---|---|---|---|---|
| $-C_3H_7$-n | H | H | H | H | $-C(CH_3)_3$ | J |
| $-C_4H_9$-n | " | " | " | " | $-C(CH_3)_3$ | Br |
| $-C_5H_{11}$-n | " | " | " | " | $-C(CH_3)_3$ | Br |
| $-C_6H_{13}$-n | " | " | " | " | $-C(CH_3)_3$ | Cl |
| $-C_3H_7$-iso | " | " | " | " | $-C(CH_3)_3$ | Cl |
| $-C_4H_9$-iso | " | " | " | " | $-C(CH_3)_3$ | Br |
| $-C_4H_9$-tert. | " | " | " | " | $-C(CH_3)_3$ | Cl |
| $-CH_2-$ 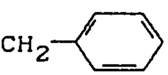 | " | " | " | " | $-CH_3$ | Br |
| $-CH_2-CH_2-$ 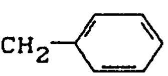 | " | " | " | " | $-CH_3$ | Cl |

<u>Tabelle 2</u> (Fortsetzung)

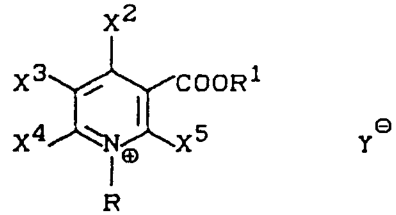

(Ib)

| R | $X^5$ | $X^2$ | $X^3$ | $X^4$ | $R^1$ | Y |
|---|---|---|---|---|---|---|
| 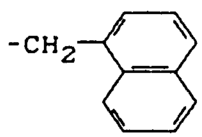 | H | H | H | H | $-CH_3$ | Cl |
| 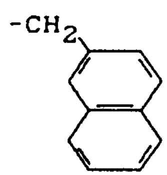 | " | " | " | " | $-CH_3$ | Cl |
| $-C_2H_5$ | " | " | " | " | $-C_2H_5$ | J |
| $-C_2H_5$ | " | " | " | " | $-C_3H_7$-n | J |
| $-C_2H_5$ | " | " | " | " | $-C_3H_7$-iso | J |
| $-C_2H_5$ | " | " | " | " | $-C_4H_9$-n | J |
| $-C_2H_5$ | " | " | " | " | $-C_4H_9$-sek | J |

Tabelle 2 (Fortsetzung)

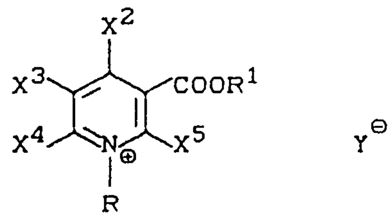

(Ib)

| R | $X^5$ | $X^2$ | $X^3$ | $X^4$ | $R^1$ | Y |
|---|---|---|---|---|---|---|
| $-C_2H_5$ | H | H | H | H | $-C_4H_9$-iso | J |
| $-C_2H_5$ | " | " | " | " | $-C_4H_9$-tert | J |
| $-C_2H_5$ | H | H | H | H | 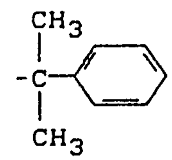 | J |
| $-C_2H_5$ | " | " | " | " | 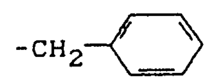 | J |
| $-C_2H_5$ | " | " | " | " | 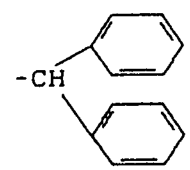 | J |

Tabelle 2 (Fortsetzung)

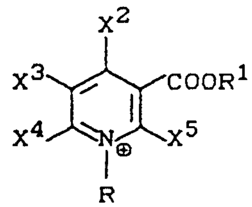

$Y^{\ominus}$

(Ib)

| R | $X^5$ | $X^2$ | $X^3$ | $X^4$ | $R^1$ | Y |
|---|---|---|---|---|---|---|
| $-C_2H_5$ | H | H | H | H | 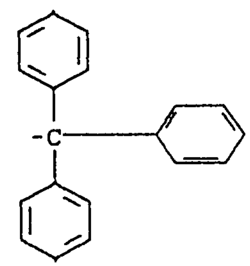 | J |
| $-C_2H_5$ | " | " | " | " | $-CH_3$ | $C_2H_5OSO_3-$ |
| $-C_2H_5$ | " | " | " | " | $-CH_3$ | 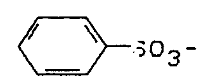 |
| $-C_2H_5$ | " | " | " | " | $-CH_3$ | $CH_3-COO-$ |
| $-C_2H_5$ | " | " | " | " | $-CH_3$ | 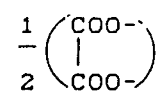 |

Tabelle 2 (Fortsetzung)

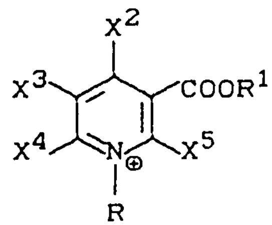

$Y^{\ominus}$ (Ib)

| R | $X^5$ | $X^2$ | $X^3$ | $X^4$ | $R^1$ | Y |
|---|---|---|---|---|---|---|
| $-C_2H_5$ | H | H | H | H | $-CH_3$ | $\frac{1}{2}$ $SO_4$ |
| $-C_2H_5$ | " | " | " | " | $-CH_3$ | $HSO_4^-$ |
| $-C_2H_5$ | " | " | " | " | $-CH_3$ | $NO_3$ |
| $-C_2H_5$ | " | " | " | " | $-CH_3$ | $BF_4$ |

Tabelle 2 (Fortsetzung)

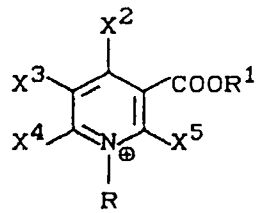

$Y^{\ominus}$ (Ib)

| R | $X^5$ | $X^2$ | $X^3$ | $X^4$ | $R^1$ | Y |
|---|---|---|---|---|---|---|
| $-CH_3$ | $CH_3$ | H | H | H | $-CH_3$ | J |
| $-CH_3$ | H | $CH_3$ | H | H | $-C_2H_5$ | J |
| $-CH_3$ | H | H | $CH_3$ | H | $-CH_3$ | $CH_3OSO_3$ |
| $-CH_3$ | H | H | H | $CH_3$ | $-C_2H_5$ | $CH_3OSO_3$ |
| $-CH_3$ | $CH_3$ | $CH_3$ | H | H | $-C_2H_5$ | $CH_3OSO_3$ |
| $-CH_3$ | $CH_3$ | H | $CH_3$ | H | $-CH_3$ | J |
| $-CH_3$ | $CH_3$ | H | H | $CH_3$ | $-CH_3$ | J |
| $-CH_3$ | H | $CH_3$ | $CH_3$ | H | $-CH_3$ | J |
| $-CH_3$ | H | $CH_3$ | H | $CH_3$ | $-CH_3$ | J |

<u>Tabelle 2</u> (Fortsetzung)

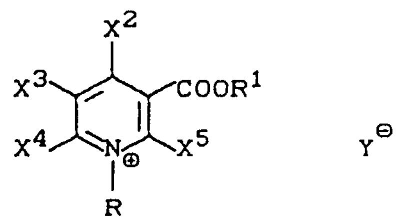

(Ib)

| R | $X^5$ | $X^2$ | $X^3$ | $X^4$ | $R^1$ | Y |
|---|---|---|---|---|---|---|
| $-CH_3$ | H | H | $CH_3$ | $CH_3$ | $-CH_3$ | J |
| $-CH_3$ | $CH_3$ | $CH_3$ | H | $CH_3$ | $-C(CH_3)_3$ | J |
| $-CH_3$ | $CH_3$ | H | H | H | $-C_2H_5$ | J |
| $-CH_3$ | H | $CH_3$ | H | H | $-CH_3$ | J |

<u>Tabelle 2</u> (Fortsetzung)

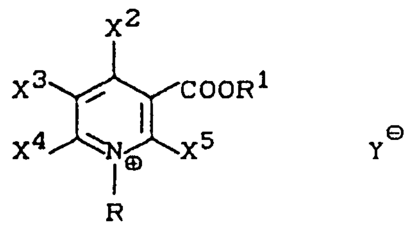

(Ib)

| R | $X^5$ | $X^2$ | $X^3$ | $X^4$ | $R^1$ | Y |
|---|---|---|---|---|---|---|
| $-CH_3$ | $CH_3$ | H | $CH_3$ | H | $-C_2H_5$ | J |
| $-CH_3$ | $CH_3$ | H | H | $CH_3$ | $-C_2H_5$ | J |
| $-CH_3$ | H | $CH_3$ | $CH_3$ | H | $-C(CH_3)_3$ | J |
| $-CH_3$ | $CH_3$ | $CH_3$ | H | $CH_3$ | $-CH_3$ | $CH_3OSO_3$ |

Tabelle 2 (Fortsetzung)

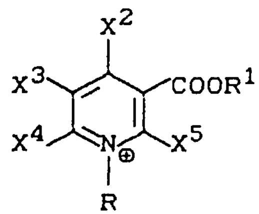

$Y^{\ominus}$

(Ib)

| R | $X^5$ | $X^2$ | $X^3$ | $X^4$ | $R^1$ | Y |
|---|---|---|---|---|---|---|
| $-CH_3$ | $CH_3$ | $CH_3$ | $CH_3$ | $CH_3$ | $-CH_3$ | $CH_3OSO_3$ |
| $-CH_3$ | $C_2H_5$ | H | H | H | $-CH_3$ | J |
| $-CH_3$ | H | H | H | $C_2H_5$ | $-CH_3$ | J |
| $-CH_3$ | H | H | H | $C_3H_7$-n | $-CH_3$ | J |
| $-CH_3$ | Cl | H | H | H | $-CH_3$ | J |
| $-CH_3$ | H | Cl | H | H | $-CH_3$ | J |
| $-CH_3$ | H | H | Cl | H | $-CH_3$ | J |
| $-CH_3$ | H | H | H | Cl | $-CH_3$ | J |
| $-CH_3$ | Cl | Cl | H | H | $-CH_3$ | J |
| $-CH_3$ | Cl | H | Cl | H | $-CH_3$ | J |

Tabelle 2 (Fortsetzung)

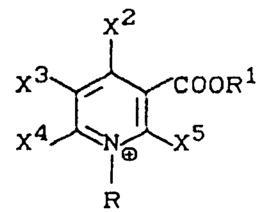

$Y^{\ominus}$

(Ib)

| R | $X^5$ | $X^2$ | $X^3$ | $X^4$ | $R^1$ | Y |
|---|---|---|---|---|---|---|
| $-CH_3$ | H | Cl | H | Cl | $-CH_3$ | J |
| $-CH_3$ | H | H | Cl | Cl | $-CH_3$ | J |
| $-CH_3$ | Br | H | H | H | $-CH_3$ | J |
| $-CH_3$ | H | H | Br | H | $-CH_3$ | J |
| $-CH_3$ | F | H | H | H | $-CH_3$ | J |
| $-CH_3$ | H | H | H | F | $-CH_3$ | J |
| $-CH_3$ | H | H | F | H | $-CH_3$ | J |
| $-CH_3$ | $CH_3$ | $CH_3$ | Cl | $CH_3$ | $-CH_3$ | J |
| $-CH_3$ | $CH_3$ | $CF_3$ | H | $CF_3$ | $-CH_3$ | J |
| $-CH_3$ | H | H | H | $CF_3$ | $-CH_3$ | J |

Tabelle 2 (Fortsetzung)

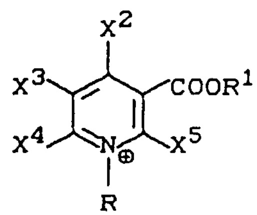

$Y^{\ominus}$

(Ib)

| R | $X^5$ | $X^2$ | $X^3$ | $X^4$ | $R^1$ | Y |
|---|---|---|---|---|---|---|
| $-CH_3$ | $CF_3$ | $CH_3$ | $CH_3$ | $CH_3$ | $-CH_3$ | J |
| $-CH_3$ | $CH_3$ | $C_2F_5$ | H | $C_2F_5$ | $-CH_3$ | J |
| $-CH_3$ | $OCF_3$ | $CH_3$ | H | $CH_3$ | $-CH_3$ | J |
| $-CH_3$ | $CH_3$ | $OCF_3$ | H | $OCF_3$ | $-CH_3$ | J |
| $-CH_3$ | $SCF_3$ | H | H | H | $-CH_3$ | J |
| $-CH_3$ | H | H | OH | H | $-CH_3$ | J |
| $-CH_3$ | H | OH | H | H | $-CH_3$ | J |
| $-CH_3$ | OH | H | H | H | $-CH_3$ | J |
| $-CH_3$ | H | SH | H | H | $-CH_3$ | J |

Tabelle 2 (Fortsetzung)

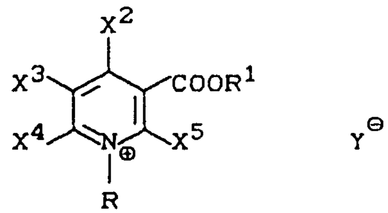

(Ib)

| R | $X^5$ | $X^2$ | $X^3$ | $X^4$ | $R^1$ | Y |
|---|---|---|---|---|---|---|
| $-CH_3$ | H | H | SH | H | $-CH_3$ | J |
| $-CH_3$ | SH | H | H | H | $-CH_3$ | J |
| $-CH_3$ | $NH_2$ | H | H | H | $-CH_3$ | J |
| $-CH_3$ | H | H | $NH_2$ | H | $-CH_3$ | J |
| $-CH_3$ | H | H | $OCH_3$ | H | $-CH_3$ | J |
| $-CH_3$ | H | $OCH_3$ | H | H | $-CH_3$ | J |
| $-CH_3$ | $OCH_3$ | H | H | H | $-CH_3$ | J |
| $-CH_3$ | $OC_2H_5$ | H | H | H | $-CH_3$ | J |
| $-CH_3$ | H | $SCH_3$ | H | H | $-CH_3$ | J |
| $-CH_3$ | H | H | $SCH_3$ | H | $-CH_3$ | J |

Tabelle 2 (Fortsetzung)

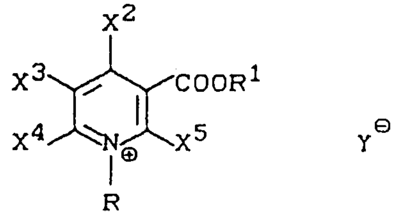

(Ib)

| R | $X^5$ | $X^2$ | $X^3$ | $X^4$ | $R^1$ | Y |
|---|---|---|---|---|---|---|
| $-CH_3$ | $SC_2H_5$ | H | H | H | $-CH_3$ | J |
| $-CH_3$ | $NH-CH_3$ | H | H | H | $-CH_3$ | J |
| $-CH_3$ | $N(CH_3)_2$ | H | H | H | $-CH_3$ | J |
| $-CH_3$ | H | H | $N(C_2H_5)_2$ | H | $-CH_3$ | J |
| $-CH_3$ | H | H | H | NH— 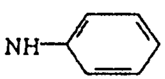 | $-CH_3$ | J |
| $-CH_3$ | $NH-CH_2$ 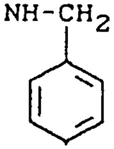 | H | H | H | $-CH_3$ | J |
| $-CH_3$ | H | 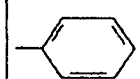 | H | 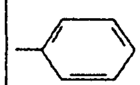 | $-CH_3$ | J |
| $-CH_3$ | 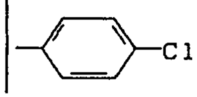 Cl | $CH_3$ | H | $CH_3$ | $-CH_3$ | J |

Tabelle 2 (Fortsetzung)

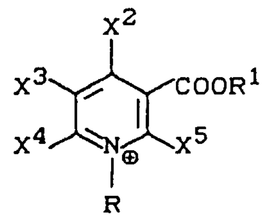

(Ib)

$Y^{\ominus}$

| R | $X^5$ | $X^2$ | $X^3$ | $X^4$ | $R^1$ | Y |
|---|---|---|---|---|---|---|
| $-CH_3$ | 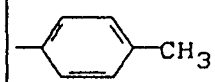 | H | H | 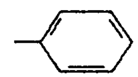 | $-CH_3$ | J |
| $-CH_3$ | 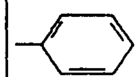 | $CH_3$ | H | 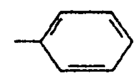 | $-CH_3$ | J |
| $-C_2H_5$ | $CH_3$ | H | H | H | $-CH_3$ | J |
| $-C_2H_5$ | H | $CH_3$ | H | H | $-C_2H_5$ | J |
| $-C_2H5$ | H | H | $CH_3$ | H | $-CH_3$ | $C_2H_5OSO_3$ |
| $-C_2H_5$ | H | H | H | $CH_3$ | $-C_2H_5$ | $C_2H_5OSO_3$ |
| $-C_2H_5$ | $CH_3$ | $CH_3$ | H | H | $-C_2H_5$ | $C_2H_5OSO_3$ |
| $-C_2H_5$ | $CH_3$ | H | $CH_3$ | H | $-CH_3$ | J |
| $-C_2H_5$ | $CH_3$ | H | H | $CH_3$ | $-CH_3$ | J |

Tabelle 2 (Fortsetzung)

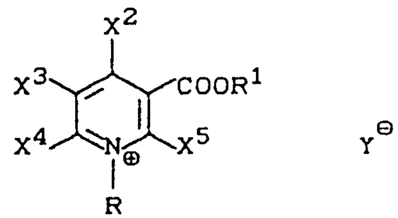

(Ib)

| R | $X^5$ | $X^2$ | $X^3$ | $X^4$ | $R^1$ | Y |
|---|---|---|---|---|---|---|
| $-C_2H_5$ | H | $CH_3$ | $CH_3$ | H | $-CH_3$ | J |
| $-C_2H_5$ | H | $CH_3$ | H | $CH_3$ | $-CH_3$ | J |
| $-C_2H_5$ | H | H | $CH_3$ | $CH_3$ | $-CH_3$ | J |
| $-C_2H_5$ | $CH_3$ | $CH_3$ | H | $CH_3$ | $-C(CH_3)_3$ | J |
| $-C_2H_5$ | Cl | H | H | H | $-CH_3$ | J |

Tabelle 2 (Fortsetzung)

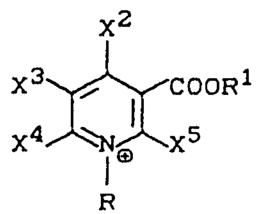

$Y^{\ominus}$

(Ib)

| R | $X^5$ | $X^2$ | $X^3$ | $X^4$ | $R^1$ | Y |
|---|---|---|---|---|---|---|
| $-C_2H_5$ | H | Cl | H | H | $-CH_3$ | J |
| $-C_2H_5$ | H | H | Cl | H | $-CH_3$ | J |
| $-C_2H_5$ | H | H | H | Cl | $-CH_3$ | J |
| $-C_2H_5$ | Cl | Cl | H | H | $-CH_3$ | J |
| $-C_2H_5$ | Cl | H | Cl | H | $-CH_3$ | J |
| $-C_2H_5$ | H | Cl | H | Cl | $-CH_3$ | J |
| $-C_2H_5$ | H | H | Cl | Cl | $-CH_3$ | J |
| $-C_2H_5$ | Br | H | H | H | $-CH_3$ | J |
| $-C_2H_5$ | H | H | Br | H | $-CH_3$ | J |
| $-C_2H_5$ | F | H | H | H | $-CH_3$ | J |

Tabelle 2 (Fortsetzung)

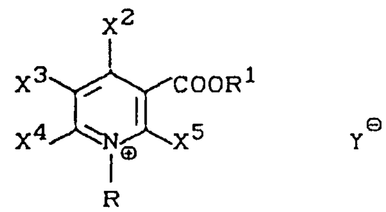

(Ib)

| R | $X^5$ | $X^2$ | $X^3$ | $X^4$ | $R^1$ | Y |
|---|---|---|---|---|---|---|
| $-C_2H_5$ | H | H | H | F | $-CH_3$ | J |
| $-C_2H_5$ | H | H | F | H | $-CH_3$ | J |
| $-C_2H_5$ | $CH_3$ | $CH_3$ | Cl | $CH_3$ | $-CH_3$ | J |
| $-C_2H_5$ | $CH_3$ | $CF_3$ | H | $CF_3$ | $-CH_3$ | J |
| $-C_2H_5$ | H | H | H | $CF_3$ | $-CH_3$ | J |
| $-C_2H_5$ | $CF_3$ | $CH_3$ | $CH_3$ | $CH_3$ | $-CH_3$ | J |
| $-C_2H_5$ | $CH_3$ | $C_2F_5$ | H | $C_2F_5$ | $-CH_3$ | J |
| $-C_2H_5$ | $OCF_3$ | $CH_3$ | H | $CH_3$ | $-CH_3$ | J |
| $-C_2H_5$ | $CH_3$ | $OCF_3$ | H | $OCF_3$ | $-CH_3$ | J |
| $-C_2H_5$ | $SCF_3$ | H | H | H | $-CH_3$ | J |

Tabelle 2 (Fortsetzung)

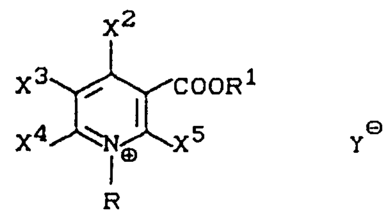

(Ib)

| R | $X^5$ | $X^2$ | $X^3$ | $X^4$ | $R^1$ | Y |
|---|---|---|---|---|---|---|
| $-C_2H_5$ | H | H | OH | H | $-CH_3$ | J |
| $-C_2H_5$ | H | OH | H | H | $-CH_3$ | J |
| $-C_2H_5$ | OH | H | H | H | $-CH_3$ | J |
| $-C_2H_5$ | H | SH | H | H | $-CH_3$ | J |
| $-C_2H_5$ | H | H | SH | H | $-CH_3$ | J |
| $-C_2H_5$ | SH | H | H | H | $-CH_3$ | J |
| $-C_2H_5$ | $NH_2$ | H | H | H | $-CH_3$ | J |
| $-C_2H_5$ | H | H | $NH_2$ | H | $-CH_3$ | J |
| $-C_2H_5$ | H | H | $OCH_3$ | H | $-CH_3$ | J |
| $-C_2H_5$ | H | $OCH_3$ | H | H | $-CH_3$ | J |

Tabelle 2 (Fortsetzung)

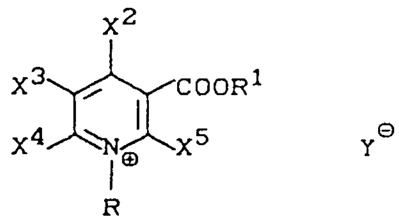

(Ib)

| R | $X^5$ | $X^2$ | $X^3$ | $X^4$ | $R^1$ | Y |
|---|---|---|---|---|---|---|
| $-C_2H_5$ | $OCH_3$ | H | H | H | $-CH_3$ | |
| $-C_2H_5$ | $OC_2H_5$ | H | H | H | $-CH_3$ | J |
| $-C_2H_5$ | H | $SCH_3$ | H | H | $-CH_3$ | J |
| $-C_2H_5$ | H | H | $SCH_3$ | H | $-CH_3$ | J |
| $-C_2H_5$ | $SC_2H_5$ | H | H | H | $-CH_3$ | J |
| $-C_2H_5$ | $NH-CH_3$ | H | H | H | $-CH_3$ | J |
| $-C_2H_5$ | $N(CH_3)_2$ | H | H | H | $-CH_3$ | J |
| $-C_2H_5$ | H | H | $N(C_2H_5)_2$ | H | $-CH_3$ | J |
| $-C_2H_5$ | H | H | H | NH- 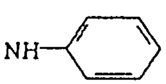 | $-CH_3$ | J |

Tabelle 2 (Fortsetzung)

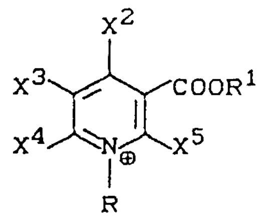

$Y^{\ominus}$

(Ib)

| R | $X^5$ | $X^2$ | $X^3$ | $X^4$ | $R^1$ | Y |
|---|---|---|---|---|---|---|
| $-C_2H_5$ | $-NH-CH_2$ 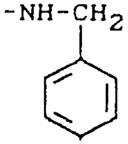 | H | H | H | $-CH_3$ | J |
| $-C_2H_5$ | H | 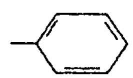 | H | 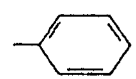 | $-CH_3$ | J |
| $-C_2H_5$ | 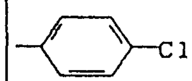 | $CH_3$ | H | $CH_3$ | $-CH_3$ | J |
| $-C_2H_5$ | 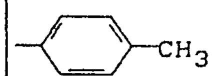 | H | H | 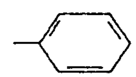 | $-CH_3$ | J |
| $-C_2H_5$ | 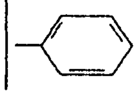 | $CH_3$ | H | 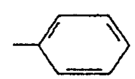 | $-CH_3$ | J |

Tabelle 2 (Fortsetzung)

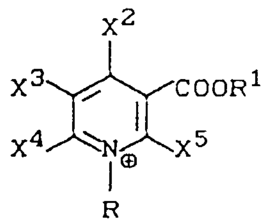

$Y^{\ominus}$

(Ib)

| R | $X^5$ | $X^2$ | $X^3$ | $X^4$ | $R^1$ | Y |
|---|---|---|---|---|---|---|
| $-C_3H_7-n$ | $CH_3$ | H | H | H | $-CH_3$ | J |
| $-C_4H_9-n$ | H | $CH_3$ | H | H | $-C_2H_5$ | Br |
| $-C_3H_7-n$ | H | Cl | H | Cl | $-C_3H_7-n$ | Br |
| $-C_6H_{13}-n$ | H | H | Cl | Cl | $-CH_3$ | Cl |
| 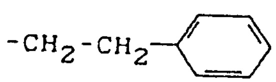 | H | H | H | F | $-CH_3$ | Cl |
| 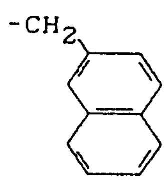 | $CF_3$ | $CH_3$ | $CH_3$ | $CH_3$ | $-CH_3$ | Cl |

Tabelle 2 (Fortsetzung)

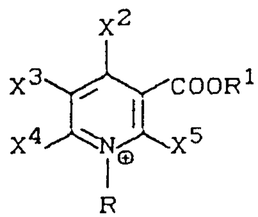

$Y^{\ominus}$

(Ib)

| R | $X^5$ | $X^2$ | $X^3$ | $X^4$ | $R^1$ | Y |
|---|---|---|---|---|---|---|
| $-C(CH_3)_3$ | H | SH | H | H | $-CH_3$ | Cl |
| $-C_4H_9$-n | H | H | $NH_2$ | H | $-C_2H_5$ | Br |
| $-C_5H_{11}$-n | $NH-CH_3$ | H | H | H | $-CH_3$ | Br |
| $-C_6H_{13}$-n | 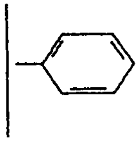 | $CH_3$ | H | 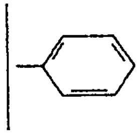 | $-CH_3$ | Cl |

Tabelle 3

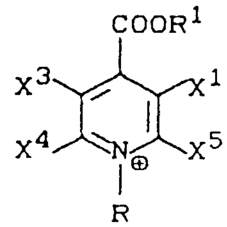

$Y^{\ominus}$

(Ic)

| R | $X^5$ | $X^1$ | $X^3$ | $X^4$ | $R^1$ | Y |
|---|---|---|---|---|---|---|
| $-C_3H_7$-n | H | H | H | H | $CH_3$ | I |
| $-C_4H_9$-n | " | " | " | " | " | Br |
| $-C_5H_{11}$-n | " | " | " | " | " | Br |
| $-C_6H_{13}$-n | " | " | " | " | " | Cl |
| $-C_3H_7$-iso | " | " | " | " | " | Cl |
| $-C_4H_9$-iso | " | " | " | " | " | Br |
| $-C_3H_7$-n | " | " | " | " | $-C(CH_3)_3$ | J |
| $-C_4H_9$-n | " | " | " | " | $-C(CH_3)_3$ | Br |
| $-C_5H_{11}$-n | " | " | " | " | $-C(CH_3)_3$ | Br |
| $-C_6H_{13}$-n | " | " | " | " | $-C(CH_3)_3$ | Cl |
| $-C_3H_7$-iso | " | " | " | " | $-C(CH_3)_3$ | Cl |
| $-C_4H_9$-iso | " | " | " | " | $-C(CH_3)_3$ | Br |

Tabelle 3 (Fortsetzung)

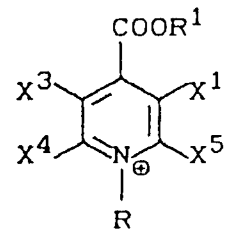

$Y^{\ominus}$

(Ic)

| R | $X^5$ | $X^1$ | $X^3$ | $X^4$ | $R^1$ | Y |
|---|---|---|---|---|---|---|
| $-C_2H_5$ | H | H | H | H | $-CH_3$ | Cl |
| $-C_2H_5$ | " | " | " | " | $-CH_3$ | J |
| $-C_2H_5$ | " | " | " | " | $-C_3H_7$-iso | J |
| $-C_2H_5$ | " | " | " | " | $-C_4H_9$-n | J |
| $-C_2H_5$ | " | " | " | " | $-C_4H_9$-sek. | J |
| $-C_2H_5$ | " | " | " | " | $-C_4H_9$-iso | J |
| $-C_2H_5$ | " | " | " | " | $-C_4H_9$-tert | J |

Tabelle 3 (Fortsetzung)

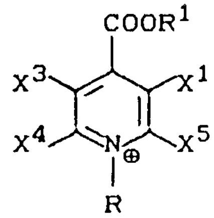

$Y^{\ominus}$

(Ic)

| R | $X^5$ | $X^1$ | $X^3$ | $X^4$ | $R^1$ | Y |
|---|---|---|---|---|---|---|
| $-C_2H_5$ | H | H | H | H | 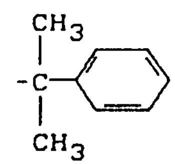 | J |
| $-C_2H_5$ | " | " | " | " | 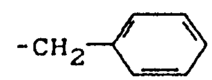 | J |
| $-C_2H_5$ | " | " | " | " | 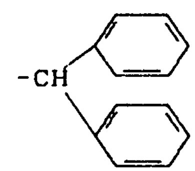 | J |
| $-C_2H_5$ | " | " | " | " | 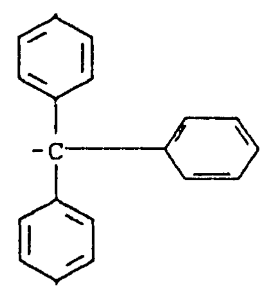 | J |

Tabelle 3 (Fortsetzung)

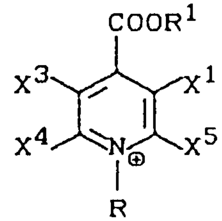 $Y^{\ominus}$ (Ic)

| R | $X^5$ | $X^1$ | $X^3$ | $X^4$ | $R^1$ | Y |
|---|---|---|---|---|---|---|
| $-C_2H_5$ | H | H | H | H | $-CH_3$ | $C_2H_5OSO_3^-$ |
| $-C_2H_5$ | " | " | " | " | $-CH_3$ | 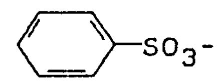 |
| $-C_2H_5$ | " | " | " | " | $-CH_3$ | $CH_3-COO-$ |
| $-C_2H_5$ | " | " | " | " | $-CH_3$ | 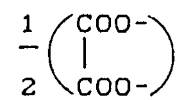 |
| $-C_2H_5$ | H | H | H | H | $-CH_3$ | $\frac{1}{2}$ $SO_4$ |
| $-C_2H_5$ | " | " | " | " | $-CH_3$ | $HSO_4^-$ |
| $-C_2H_5$ | " | " | " | " | $-CH_3$ | $NO_3$ |
| $-C_2H_5$ | " | " | " | " | $-CH_3$ | $BF_4$ |

Tabelle 3 (Fortsetzung)

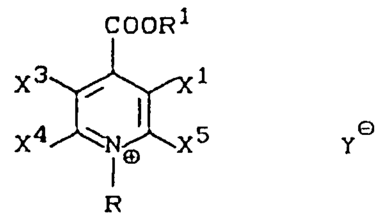

(Ic)

| R | $X^5$ | $X^1$ | $X^3$ | $X^4$ | $R^1$ | Y |
|---|---|---|---|---|---|---|
| $-CH_3$ | $CH_3$ | H | H | H | $-CH_3$ | J |
| $-CH_3$ | H | $CH_3$ | H | H | $-C_2H_5$ | J |
| $-CH_3$ | H | H | $CH_3$ | H | $-CH_3$ | $CH_3OSO_3$ |
| $-CH_3$ | H | H | H | $CH_3$ | $-C_2H_5$ | $CH_3OSO_3$ |
| $-CH_3$ | $CH_3$ | $CH_3$ | H | H | $-C_2H_5$ | $CH_3OSO_3$ |
| $-CH_3$ | $CH_3$ | H | $CH_3$ | H | $-CH_3$ | J |
| $-CH_3$ | $CH_3$ | H | H | $CH_3$ | $-CH_3$ | J |
| $-CH_3$ | H | $CH_3$ | $CH_3$ | H | $-CH_3$ | J |
| $-CH_3$ | H | $CH_3$ | H | $CH_3$ | $-CH_3$ | J |

<u>Tabelle 3</u> (Fortsetzung)

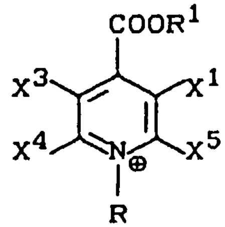 $Y^{\ominus}$ (Ic)

| R | $X^5$ | $X^1$ | $X^3$ | $X^4$ | $R^1$ | Y |
|---|---|---|---|---|---|---|
| $-CH_3$ | H | H | $CH_3$ | $CH_3$ | $-CH_3$ | J |
| $-CH_3$ | $CH_3$ | $CH_3$ | H | $CH_3$ | $-C(CH_3)_3$ | J |
| $-CH_3$ | $CH_3$ | H | H | H | $-C_2H_5$ | J |
| $-CH_3$ | H | $CH_3$ | H | H | $-CH_3$ | J |
| $-CH_3$ | $CH_3$ | H | $CH_3$ | H | $-C_2H_5$ | J |
| $-CH_3$ | $CH_3$ | H | H | $CH_3$ | $-C_2H_5$ | J |
| $-CH_3$ | H | $CH_3$ | $CH_3$ | H | $-C(CH_3)_3$ | J |
| $-CH_3$ | $CH_3$ | $CH_3$ | H | $CH_3$ | $-CH_3$ | $CH_3OSO_3$ |

<u>Tabelle 3</u> (Fortsetzung)

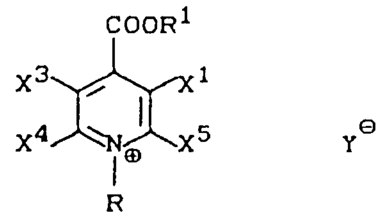

(Ic)

| R | $X^5$ | $X^1$ | $X^3$ | $X^4$ | $R^1$ | Y |
|---|---|---|---|---|---|---|
| $-CH_3$ | $CH_3$ | $CH_3$ | $CH_3$ | $CH_3$ | $-CH_3$ | $CH_3OSO_3$ |
| $-CH_3$ | $C_2H_5$ | H | H | H | $-CH_3$ | J |
| $-CH_3$ | H | H | H | $C_2H_5$ | $-CH_3$ | J |
| $-CH_3$ | H | H | H | $C_3H_7$-n | $-CH_3$ | J |
| $-CH_3$ | Cl | H | H | H | $-CH_3$ | J |
| $-CH_3$ | H | Cl | H | H | $-CH_3$ | J |
| $-CH_3$ | H | H | Cl | H | $-CH_3$ | J |
| $-CH_3$ | H | H | H | Cl | $-CH_3$ | J |
| $-CH_3$ | Cl | Cl | H | H | $-CH_3$ | J |
| $-CH_3$ | Cl | H | Cl | H | $-CH_3$ | J |

Tabelle 3 (Fortsetzung)

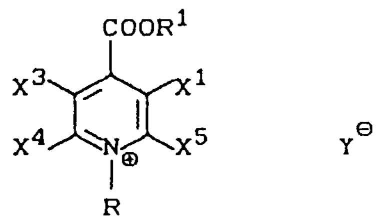

(Ic)

| R | $X^5$ | $X^1$ | $X^3$ | $X^4$ | $R^1$ | Y |
|---|---|---|---|---|---|---|
| $-CH_3$ | H | Cl | H | Cl | $-CH_3$ | J |
| $-CH_3$ | H | H | Cl | Cl | $-CH_3$ | J |
| $-CH_3$ | Br | H | H | H | $-CH_3$ | J |
| $-CH_3$ | H | H | Br | H | $-CH_3$ | J |
| $-CH_3$ | F | H | H | H | $-CH_3$ | J |
| $-CH_3$ | H | H | H | F | $-CH_3$ | J |
| $-CH_3$ | H | H | F | H | $-CH_3$ | J |
| $-CH_3$ | $CH_3$ | $CH_3$ | Cl | $CH_3$ | $-CH_3$ | J |
| $-CH_3$ | $CH_3$ | $CF_3$ | H | $CF_3$ | $-CH_3$ | J |
| $-CH_3$ | H | H | H | $CF_3$ | $-CH_3$ | J |

Tabelle 3 (Fortsetzung)

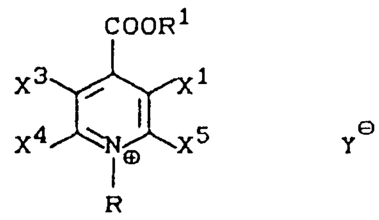

(Ic)

| R | $X^5$ | $X^1$ | $X^3$ | $X^4$ | $R^1$ | Y |
|---|---|---|---|---|---|---|
| $-CH_3$ | $CF_3$ | $CH_3$ | $CH_3$ | $CH_3$ | $-CH_3$ | J |
| $-CH_3$ | $CH_3$ | $C_2F_5$ | H | $C_2F_5$ | $-CH_3$ | J |
| $-CH_3$ | $OCF_3$ | $CH_3$ | H | $CH_3$ | $-CH_3$ | J |
| $-CH_3$ | $CH_3$ | $OCF_3$ | H | $OCF_3$ | $-CH_3$ | J |
| $-CH_3$ | $SCF_3$ | H | H | H | $-CH_3$ | J |
| $-CH_3$ | H | H | OH | H | $-CH_3$ | J |
| $-CH_3$ | H | OH | H | H | $-CH_3$ | J |
| $-CH_3$ | OH | H | H | H | $-CH_3$ | J |
| $-CH_3$ | H | SH | H | H | $-CH_3$ | J |

Tabelle 3 (Fortsetzung)

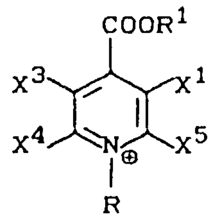

$Y^{\ominus}$

(Ic)

| R | $X^5$ | $X^1$ | $X^3$ | $X^4$ | $R^1$ | Y |
|---|---|---|---|---|---|---|
| $-CH_3$ | H | H | SH | H | $-CH_3$ | J |
| $-CH_3$ | SH | H | H | H | $-CH_3$ | J |
| $-CH_3$ | $NH_2$ | H | H | H | $-CH_3$ | J |
| $-CH_3$ | H | H | $NH_2$ | H | $-CH_3$ | J |
| $-CH_3$ | H | H | $OCH_3$ | H | $-CH_3$ | J |
| $-CH_3$ | H | $OCH_3$ | H | H | $-CH_3$ | J |
| $-CH_3$ | $OCH_3$ | H | H | H | $-CH_3$ | J |
| $-CH_3$ | $OC_2H_5$ | H | H | H | $-CH_3$ | J |
| $-CH_3$ | H | $SCH_3$ | H | H | $-CH_3$ | J |
| $-CH_3$ | H | H | $SCH_3$ | H | $-CH_3$ | J |

Tabelle 3 (Fortsetzung)

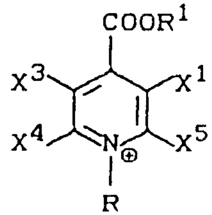

$Y^{\ominus}$

(Ic)

| R | $X^5$ | $X^1$ | $X^3$ | $X^4$ | $R^1$ | Y |
|---|---|---|---|---|---|---|
| $-CH_3$ | $SC_2H_5$ | H | H | H | $-CH_3$ | J |
| $-CH_3$ | $NH-CH_3$ | H | H | H | $-CH_3$ | J |
| $-CH_3$ | $N(CH_3)_2$ | H | H | H | $-CH_3$ | J |
| $-CH_3$ | H | H | $N(C_2H_5)_2$ | H | $-CH_3$ | J |
| $-CH_3$ | H | H | H | NH 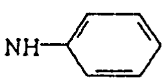 | $-CH_3$ | J |
| $-CH_3$ | $NH-CH_2$ 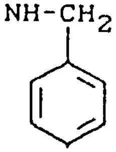 | H | H | H | $-CH_3$ | J |
| $-CH_3$ | H | 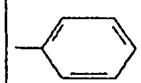 | H | 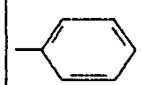 | $-CH_3$ | J |
| $-CH_3$ | 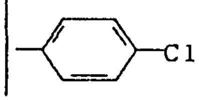 | $CH_3$ | H | $CH_3$ | $-CH_3$ | J |

Tabelle 3 (Fortsetzung)

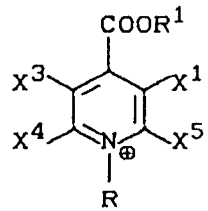

$Y^{\ominus}$

(Ic)

| R | $X^5$ | $X^1$ | $X^3$ | $X^4$ | $R^1$ | Y |
|---|---|---|---|---|---|---|
| $-CH_3$ | 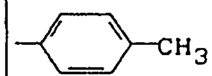 | H | H | 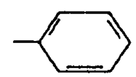 | $-CH_3$ | J |
| $-CH_3$ | 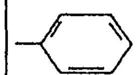 | $CH_3$ | H | 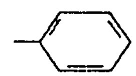 | $-CH_3$ | J |
| $-C_2H_5$ | $CH_3$ | H | H | H | $-CH_3$ | J |
| $-C_2H_5$ | H | $CH_3$ | H | H | $-C_2H_5$ | J |
| $-C_2H5$ | H | H | $CH_3$ | H | $-CH_3$ | $C_2H_5OSO_3$ |
| $-C_2H_5$ | H | H | H | $CH_3$ | $-C_2H_5$ | $C_2H_5OSO_3$ |
| $-C_2H_5$ | $CH_3$ | $CH_3$ | H | H | $-C_2H_5$ | $C_2H_5OSO_3$ |
| $-C_2H_5$ | $CH_3$ | H | $CH_3$ | H | $-CH_3$ | J |
| $-C_2H_5$ | $CH_3$ | H | H | $CH_3$ | $-CH_3$ | J |

Tabelle 3 (Fortsetzung)

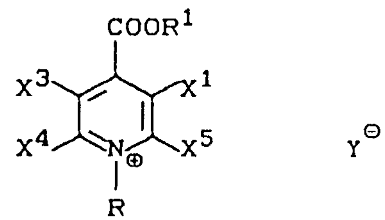

(Ic)

| R | $X^5$ | $X^2$ | $X^3$ | $X^4$ | $R^1$ | Y |
|---|---|---|---|---|---|---|
| $-C_2H_5$ | H | $CH_3$ | $CH_3$ | H | $-CH_3$ | J |
| $-C_2H_5$ | H | $CH_3$ | H | $CH_3$ | $-CH_3$ | J |
| $-C_2H_5$ | H | H | $CH_3$ | $CH_3$ | $-CH_3$ | J |
| $-C_2H_5$ | $CH_3$ | $CH_3$ | H | $CH_3$ | $-C(CH_3)_3$ | J |
| $-C_2H_5$ | Cl | H | H | H | $-CH_3$ | J |

Tabelle 3 (Fortsetzung)

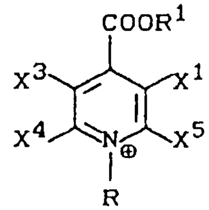

$Y^{\ominus}$

(Ic)

| R | $X^5$ | $X^1$ | $X^3$ | $X^4$ | $R^1$ | Y |
|---|---|---|---|---|---|---|
| $-C_2H_5$ | H | Cl | H | H | $-CH_3$ | J |
| $-C_2H_5$ | H | H | Cl | H | $-CH_3$ | J |
| $-C_2H_5$ | H | H | H | Cl | $-CH_3$ | J |
| $-C_2H_5$ | Cl | Cl | H | H | $-CH_3$ | J |
| $-C_2H_5$ | Cl | H | Cl | H | $-CH_3$ | J |
| $-C_2H_5$ | H | Cl | H | Cl | $-CH_3$ | J |
| $-C_2H_5$ | H | H | Cl | Cl | $-CH_3$ | J |
| $-C_2H_5$ | Br | H | H | H | $-CH_3$ | J |
| $-C_2H_5$ | H | H | Br | H | $-CH_3$ | J |
| $-C_2H_5$ | F | H | H | H | $-CH_3$ | J |

Tabelle 3 (Fortsetzung)

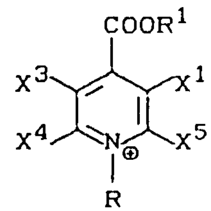

$Y^{\ominus}$

(Ic)

| R | $X^5$ | $X^1$ | $X^3$ | $X^4$ | $R^1$ | Y |
|---|---|---|---|---|---|---|
| $-C_2H_5$ | H | H | H | F | $-CH_3$ | J |
| $-C_2H_5$ | H | H | F | H | $-CH_3$ | J |
| $-C_2H_5$ | $CH_3$ | $CH_3$ | Cl | $CH_3$ | $-CH_3$ | J |
| $-C_2H_5$ | $CH_3$ | $CF_3$ | H | $CF_3$ | $-CH_3$ | J |
| $-C_2H_5$ | H | H | H | $CF_3$ | $-CH_3$ | J |
| $-C_2H_5$ | $CF_3$ | $CH_3$ | $CH_3$ | $CH_3$ | $-CH_3$ | J |
| $-C_2H_5$ | $CH_3$ | $C_2F_5$ | H | $C_2F_5$ | $-CH_3$ | J |
| $-C_2H_5$ | $OCF_3$ | $CH_3$ | H | $CH_3$ | $-CH_3$ | J |
| $-C_2H_5$ | $CH_3$ | $OCF_3$ | H | $OCF_3$ | $-CH_3$ | J |
| $-C_2H_5$ | $SCF_3$ | H | H | H | $-CH_3$ | J |

<u>Tabelle 3</u> (Fortsetzung)

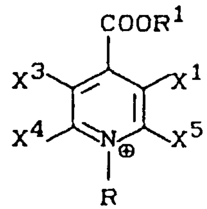

$Y^{\ominus}$

(Ic)

| R | $X^5$ | $X^1$ | $X^3$ | $X^4$ | $R^1$ | Y |
|---|---|---|---|---|---|---|
| $-C_2H_5$ | H | H | OH | H | $-CH_3$ | J |
| $-C_2H_5$ | H | OH | H | H | $-CH_3$ | J |
| $-C_2H_5$ | OH | H | H | H | $-CH_3$ | J |
| $-C_2H_5$ | H | SH | H | H | $-CH_3$ | J |
| $-C_2H_5$ | H | H | SH | H | $-CH_3$ | J |
| $-C_2H_5$ | SH | H | H | H | $-CH_3$ | J |
| $-C_2H_5$ | $NH_2$ | H | H | H | $-CH_3$ | J |
| $-C_2H_5$ | H | H | $NH_2$ | H | $-CH_3$ | J |
| $-C_2H_5$ | H | H | $OCH_3$ | H | $-CH_3$ | J |
| $-C_2H_5$ | H | $OCH_3$ | H | H | $-CH_3$ | J |

Tabelle 3 (Fortsetzung)

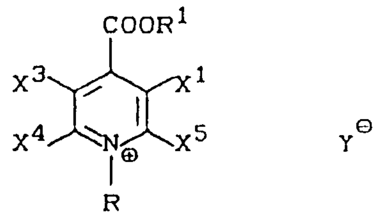

(Ic)

| R | $X^5$ | $X^1$ | $X^3$ | $X^4$ | $R^1$ | Y |
|---|---|---|---|---|---|---|
| $-C_2H_5$ | $OCH_3$ | H | H | H | $-CH_3$ | J |
| $-C_2H_5$ | $OC_2H_5$ | H | H | H | $-CH_3$ | J |
| $-C_2H_5$ | H | $SCH_3$ | H | H | $-CH_3$ | J |
| $-C_2H_5$ | H | H | $SCH_3$ | H | $-CH_3$ | J |
| $-C_2H_5$ | $SC_2H_5$ | H | H | H | $-CH_3$ | J |
| $-C_2H_5$ | $NH-CH_3$ | H | H | H | $-CH_3$ | J |
| $-C_2H_5$ | $N(CH_3)_2$ | H | H | H | $-CH_3$ | J |
| $-C_2H_5$ | H | H | $N(C_2H_5)_2$ | H | $-CH_3$ | J |
| $-C_2H_5$ | H | H | H | 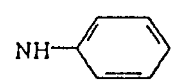 | $-CH_3$ | J |

Tabelle 3 (Fortsetzung)

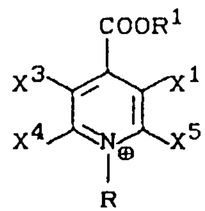

$Y^{\ominus}$

(Ic)

| R | $X^5$ | $X^1$ | $X^3$ | $X^4$ | $R^1$ | Y |
|---|---|---|---|---|---|---|
| $-C_2H_5$ | $-NH-CH_2$ 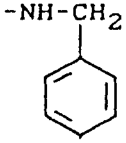 | H | H | H | $-CH_3$ | J |
| $-C_2H_5$ | H | 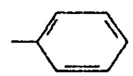 | H | 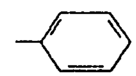 | $-CH_3$ | J |
| $-C_2H_5$ | 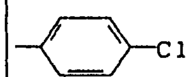 | $CH_3$ | H | $CH_3$ | $-CH_3$ | J |
| $-C_2H_5$ | 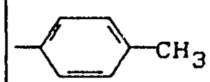 | H | H | 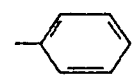 | $-CH_3$ | J |
| $-C_2H_5$ | 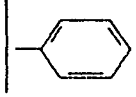 | $CH_3$ | H | 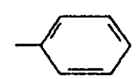 | $-CH_3$ | J |

Tabelle 3 (Fortsetzung)

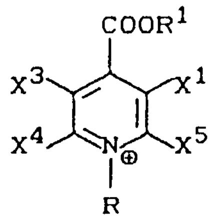

$Y^{\ominus}$

(Ic)

| R | $X^5$ | $X^1$ | $X^3$ | $X^4$ | $R^1$ | Y |
|---|---|---|---|---|---|---|
| $-C_3H_7$-n | $CH_3$ | H | H | H | $-CH_3$ | J |
| $-C_4H_9$-n | H | $CH_3$ | H | H | $-C_2H_5$ | Br |
| $-C_3H_7$-n | H | Cl | H | Cl | $-C_3H_7$-n | Br |
| $-C_6H_{13}$-n | H | H | Cl | Cl | $-CH_3$ | Cl |
| $-CH_2-CH_2-$ 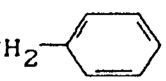 | H | H | H | F | $-CH_3$ | Cl |
| $-CH_2$ 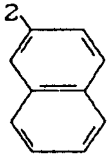 | $CF_3$ | $CH_3$ | $CH_3$ | $CH_3$ | $-CH_3$ | Cl |
| $-C(CH_3)_3$ | H | SH | H | H | $-CH_3$ | Cl |

**Tabelle 3** (Fortsetzung)

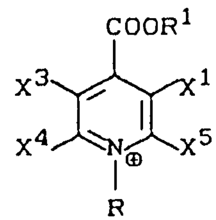

$Y^{\ominus}$

(Ic)

| R | $X^5$ | $X^1$ | $X^3$ | $X^4$ | $R^1$ | Y |
|---|---|---|---|---|---|---|
| $-C_4H_9$-n | H | H | $NH_2$ | H | $-C_2H_5$ | Br |
| $-C_5H_{11}$-n | $NH-CH_3$ | H | H | H | $-CH_3$ | Br |
| $-C_6H_{13}$-n | 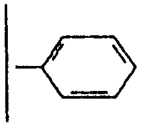 | $CH_3$ | H | 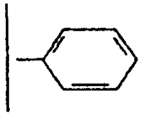 | $-CH_3$ | Cl |

Die erfindungsgemäß verwendbaren Pyridinium-Salze der Formel (I) sind teilweise bekannt (vgl. J. Org. Chem. 26, 1318-1319 (1961), Phytochemistry 23, 1225-1228 (1984) und EP-A 0 127 670). Sie lassen sich nach bekannten Verfahren herstellen. So erhält man Pyridinium-Salze der Formel (I), indem man Pyridin-Derivate der Formel

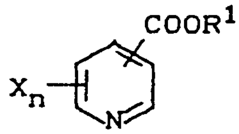

(II)

in welcher

$R^1$, X und n die oben angegebene Bedeutung haben, mit Verbindungen der Formel

$R\text{-}Y^1$ (III)

in welcher

R die oben angegebene Bedeutung hat und

$Y^1$ für Chlor, Brom, Iod, Sulfonyl oder Alkylsulfat oder den Rest der Formel $-O^{\oplus}R^6\ B^{\ominus}F_4$ steht, worin $R^6$ für Methyl oder Ethyl steht,

in Gegenwart eines Verdünnungsmittels umsetzt und gegebenenfalls anschließend die so erhaltenen Pyridinium-Salze der Formel

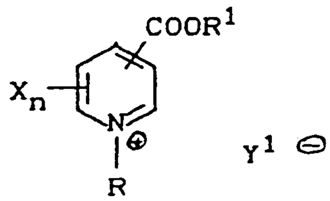

(Id)

in welcher

R, $R^1$, X, $Y^1$ und n die oben angegebene Bedeutung haben,

zum Austausch des Anions über einen Anionenaustauscher, der mit dem jeweils gewünschten Anion beladen ist, laufen läßt.

Die bei dem obigen Verfahren zur Herstellung von Pyridinium-Salzen als Ausgangsstoffe benötigten Pyridin-Derivate sind durch die Formel (II) allgemein definiert. In dieser Formel haben $R^1$, X und n vorzugsweise diejenigen Bedeutungen, die bereits im Zusammenhang mit der Beschreibung der erfindungsgemäß verwendbaren Stoffe der Formel (I) für diese Reste bzw. für diesen Index als bevorzugt genannt wurden.

Die Pyridin-Derivate der Formel (II) sind bekannt oder lassen sich nach bekannten Verfahren herstellen (vgl. JP-A 1987-161 761 und JP-A 1980-108 818).

Die bei dem obigen Verfahren zur Herstellung von Pyridinium-Salzen als Reaktionskomponenten benötigten Verbindungen sind durch die Formel (III) allgemein definiert. In dieser Formel hat R vorzugsweise diejenigen Bedeutungen, die bereits im Zusammenhang mit der Beschreibung der erfindungsgemäß verwendbaren Stoffe der Formel (I) für diesen Rest als bevorzugt genannt wurden. $Y^1$ steht vorzugsweise für Chlor, Brom, Iod, Sulfonyl oder Alkylsulfat mit 1 bis 4 Kohlenstoffatomen oder für den Rest der Formel $-O^{\oplus}R^6\ BF_4^{\ominus}$, worin $R^6$ für Methyl oder Ethyl steht.

Die Verbindungen der Formel (III) sind bekannt oder lassen sich nach bekannten Verfahren herstellen.

Als Verdünnungsmittel kommen bei dem obigen Verfahren zur Herstellung von Pyridinium-Salzen der Formel (I) alle inerten organischen Solventien in Betracht.

Vorzugsweise verwendbar sind aliphatische und aromatische Kohlenwasserstoffe, wie Pentan, Hexan, Benzol, Toluol und Xylol, chlorierte Kohlenwasserstoffe, wie Dichlormethan und Chloroform, ferner Ether, wie Diethylether, Dioxan oder Tetrahydrofuran, weiterhin Ester, wie Essigsäureethylester, ferner Nitrile, wie Acetonitril, außerdem Ketone, wie Aceton, und außerdem polare organische Lösungsmittel, wie Dimethylformamid oder Dimethylsulfoxid. In manchen Fällen kann auch Wasser, gegebenenfalls im Gemisch mit einem organischen Lösungsmittel als Verdünnungsmittel verwendet werden.

Die Reaktionstemperaturen können bei der Durchführung des obigen Verfahrens zur Herstellung von Pyridinium-Salzen innerhalb eines größeren Bereiches variiert werden. Im allgemeinen arbeitet man bei Temperaturen zwischen 0 °C und 200 °C, vorzugsweise zwischen 20 °C und 150 °C.

Bei dem obigen Verfahren zur Herstellung von Pyridinium-Salzen setzt man auf 1 Mol an Pyridinium-Derivat der Formel (II) im allgemeinen 1 bis 10 Mol, vorzugsweise 1 bis 3 Mol an einer Verbindung der

Formel (III) ein. Die Aufarbeitung erfolgt nach üblichen Methoden.

Sofern beabsichtigt ist, in den zunächst anfallenden Pyridinium-Salzen der Formel (Id) das Anion auszutauschen, so können zu diesem Zweck alle üblichen Anionenaustauscher verwendet werden, die zuvor mit dem jeweils gewünschten Anion beladen wurden. Vorzugsweise verwendbare Anionenaustauscher sind Lewatite.

Bei der Durchführung des Anionenaustausches arbeitet man im allgemeinen bei Raumtemperatur. Es ist jeweils auch möglich, bei erhöhten oder erniedrigten Temperaturen zu arbeiten.

Bei der Durchführung des Anionenaustausches kommen bei dem obigen Verfahren als Lösungsmittel, die sowohl zum Auftragen der Pyridinium-Salze als auch zur Eluierung dienen, alle für derartige Zwecke üblichen organischen Solventien sowie Wasser in Betracht. Vorzugsweise verwendbar ist Wasser, gegebenenfalls im Gemisch mit einem organischen Lösungsmittel.

Bei der Durchführung des Anionenaustausches nach dem obigen Verfahren geht man im allgemeinen so vor, daß man das Pyridinium-Salz in einer geringen Menge an Solvens gelöst auf den jeweiligen, in einer Säule befindlichen Anionenaustauscher aufträgt und dann eluiert. Aus dem Eluat lassen sich die gewünschten Verbindungen durch Abziehen des Verdünnungsmittels isolieren.

Die erfindungsgemäß verwendbaren Pyridinium-Salze weisen eine starke resistenz-induzierende Wirkung in Pflanzen auf. Sie eignen sich daher zur Erzeugung von Resistenz in Pflanzen gegen Befall durch unerwünschte Mikroorganismen.

Unter resistenz-induzierenden Stoffen sind im vorliegenden Zusammenhang solche Substanzen zu verstehen, die einerseits bei direkter Einwirkung auf die unerwünschten Mikroorganismen nur eine geringe Aktivität zeigen, andererseits aber in der Lage sind, das Abwehrsystem von Pflanzen so zu stimulieren, daß die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogone Pilze, Bakterien und Viren zu verstehen. Die erfindungsgemäß verwendbaren Stoffe können also eingesetzt werden, um in Pflanzen innerhalb eines gewissen Zeitraumes nach der Behandlung Resistenz gegen den Befall durch die genannten Schaderreger zu erzeugen. Der Zeitraum, innerhalb dessen Resistenz herbeigeführt wird, erstreckt sich im allgemeinen von 1 bis 10 Tage, vorzugsweise 1 bis 7 Tage nach der Behandlung der Pflanzen mit den Wirkstoffen.

Zu den unerwünschten Mikroorganismen im Pflanzenschutz gehören Pilze aus den Klassen der Plasmodiophoromycetes, Oomycetes, Chytridiomycetes, Zygomycetes, Ascomycetes, Basidiomycetes und Deuteromycetes.

Beispielhaft, aber nicht begrenzend seien einige Erreger von pilzlichen, bakteriellen und virösen Erkrankungen genannt, die unter die oben aufgezählten Oberbegriffe fallen:

Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae, Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotrichia;
Venturia-Arten, wie beispielsweise Venturia ineaqualis;
Pyrenophora-Arten, wie beispielsweise Pyrenophora teres oder Pyrenophora graminea (Konidienform: Dreslera sp., Syn. Helminthosporium sp.);
Cochliobolus-Arten, wie beispielsweise Cochliobolus sativus (Konidienform: Drechslera sp., Syn. Helminthosporium sp);
Uromyces-Arten, wie beispielsweise Uromyces phaseoli;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum
oder Fusarium graminearum;
Botrytis-Arten, wie beispielsweise Botrytis cinerea;
Leptosphaeria-Arten, wie beispielsweise Leptosphaeria nodorum oder Leptosphaeria tritici (Konidienform: Septoria sp.);

Leptosphaeria-Arten, wie beispielsweise Leptosphaeria maculans (Konidienform: Phoma lingam) Cerospora-Arten, wie beispielsweise Cercospora canescens oder Cercospora beticola;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Induktion von Krankheitsresistenz in Pflanzen notwendigen Konzentrationen erlaubt eine Behandlung von oberirdischen Pflanzenteilen, von Pflanz- und Saatgut und des Bodens.

Dabei lassen sich die erfindungsgemäß verwendbaren Wirkstoffe mit besonders gutem Erfolg einsetzen

- zur Verhinderung des Auftretens von Getreidekrankheiten, wie beispielsweise des Getreidemehltaus (Erysiphe graminis),
  der Streifenkrankheit der Gerste (Pyrenonhora graminea),
  der Braunfleckenkrankheit des Getreides (Cochliobolus sativus),
  der Netzfleckenkrankheit der Gerste (Pyrenophora teres),
  der Septoria-Erkrankungen des Getreides (Septoria nodorum, Sentoria tritici),
  der Fusarium-Erkrankungen (Fusarium culmorum, Fusarium graminearum),
  der Rostkrankheiten des Getreides (Puccinia recondita, Puccinia graminis, Puccinia glumarum),
- zur Verhinderung des Auftretens von Reiskrankheiten, die beispielsweise hervorgerufen werden durch Pyricularia oryzae oder Pellicularia sasakii;
- zur Verhinderung des Auftretens von Obst- und Gemüsekrankheiten, wie beispielsweise
  den Echten Mehltau an verschiedenen Wirtspflanzen (Erysiphe sp., Sphaerotheca sp., Podosphaera sp.);
  den Falschen Mehltau an verschiedenen Wirtspflanzen (Plasmopara sp., Peronospora sp., Pseudoperonospora sp., Bremia sp.);
  den Schorf an verschiedenen Wirtspflanzen (Venturia sp.);
  den Grauschimmel an verschiedenen Wirtspflanzen (Botrytis cinerea).

Neben der Resistenz-induzierenden Wirkung zeichnen sich die erfindungsgemäß verwendbaren Wirkstoffe insbesondere auch durch eine gute systemische Wirksamkeit aus.

Zu den unerwünschten Mikroorganismen im Pflanzenschutz gehören auch Viren. Als Beispiele genannt seien das Tabakmosaik-Virus, das Apfelmosaik-Virus und das Gurkenmosaik-Virus.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schaume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflachenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage; z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol,

Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungiziden, Insektiziden, Bakterziden, Akariziden, Nematiziden, Herbiziden, Schutzstoffen gegen Vogelfraß, Wuchsstoffen, Pflanzennährstoffen und Bodenstrukturverbesserungsmitteln.

Als Beispiele für Fungizide seien in diesem Zusammenhang genannt: Imazalil, Benomyl, Carbendazim, Thiophanat-methyl, Captatol, Captan, Schwefel, Triforin, Dodemorph, Tridemorph, Pyrazophos, Furalaxyl, Ethirimol, Dimethirimol, Bupirimat, Chlorothalonil, Vinclozolin, Procymidon, Iprodion, Metalaxyl, Carboxin, Oxycarboxin, Fenarimol, Nuarimol, Fenfuram, Methfuroxan, Nitrotalisopropyl, Triadimefon, Thiabendazol, Etridiazol, Triadimenol, Bitertanol, Propiconazol, Anilazin, Tebuconazol, Biloxazol, Dithianon, Binapacryl, Quinomethionat, Guazatin, Dodine, Fentin-acetat, Fentinhydroxid, Dinocap, Folpet, Dichlofluanid, Ditalimphos, Kitazin, Cycloheximid, Dichlobutrazol, Fenapanil, Ofurace, Etaconazol und Fenpropemorph.

Die Wirkstoffe können als solche, in Form ihrer Formulierungen oder den daraus durch weiteres Verdünnen bereiteten Anwendungsformen, wie gebrauchsfertige Lösungen, Emulsionen, Suspensionen, Pulver, Pasten und Granulate, angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Anwendung als Resistenzinduktoren können die Wirkstoffkonzentrationen für die Behandlung von Pflanzenteilen in den Anwendungsformen in einem größeren Bereich variiert werden. Sie liegen im allgemeinen zwischen 1 und 0,0001 Gew.%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 1,0 Gewichtsprozent, vorzugsweise von 0,0001 bis 0,02 Gewichtsprozent, am Wirkort erforderlich.

Die Herstellung und die Verwendung der erfindungsgemäßen Stoffe wird durch die folgenden Beispiele veranschaulicht.

Herstellungsbeispiele

Beispiel 1

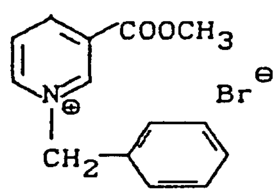

Zu einer Lösung von 22,7 g (0,165 Mol) Nicotinsäuremethylester in 250 ml Toluol werden 30,9 g (0,182 Mol) Benzylbromid gegeben. Das Reaktionsgemisch wird 16 Stunden unter Rückfluß erhitzt und dann abgekühlt. Der dabei anfallende Feststoff wird abfiltriert und getrocknet. Man erhält auf diese Weise 24,1 g (48 % der Theorie) an 1-Benzyl-3-carbomethoxy-pyridinium-bromid in Form einer Festsubstanz.

NMR-Spektrum; 400 MHz (DMSO-$d_6$ / TMS)

$\delta$ 4,0 ppm $COOCH_3$

$\delta$ 6,1 ppm

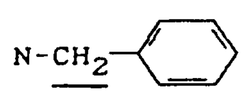

δ 8,4 - 8,5 ppm 1H-Pyridin
Nach der im Beispiel 1 angegebenen Methode werden auch die in der folgenden Tabelle 4 aufgeführten Pyridinium-Salze erhalten.

Tabelle 4

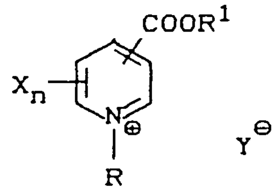

(I)

| Bsp.- | R | $-COOR^1$ | $X_n$ | Y | Physikal. Konstante |
|---|---|---|---|---|---|
| 2 | $-C_2H_5$ | $3-COOCH_3$ | - | J | NMR δ = 4,7-4,8 |
| 3 | $-CH_3$ | $4-COO-C_2H_5$ | - | J | Fp = 118° C |
| 4 | $-CH_3$ | $3-COOCH_3$ | - | J | Fp = 131° C |
| 5 | $-CH_2-$ 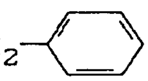 | $4-COO-C_2H_5$ | - | Br | Fp = 172° C |
| 6 | $-C_2H_5$ | $4-COO-C_2H_5$ | - | J | NMR δ = 4,7-4,8 |
| 7 | $-C_2H_5$ | $2-COO-C_2H_5$ | - | $BF_4$ | Fp=77-78° C |
| 8 | $-CH_3$ | $3-COO-CH_2$ 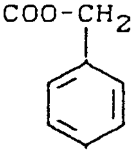 | - | J | Fp=170-172° C |
| 9 | $-CH_3$ | 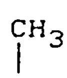 3-COO-CH 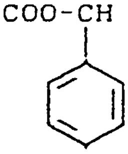 | - | J | Fp=162-164° C |
| 10 | $-CH_3$ | 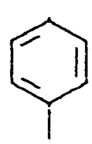 3-COO-CH 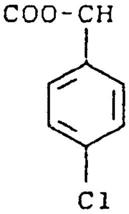 | - | J | Fp=154-156° C |

In den folgenden Verwendungsbeispielen wird die Verbindung der nachstehend angegebenen Formel als Vergleichssubstanz eingesetzt:

(A) = 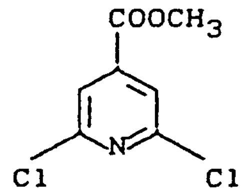

(Bekannt aus EP-A 0 268 775)

Beispiel A

Plasmopara-Test an Blattstücken von Reben / protektiv (Resistenzinduktion)

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung der Resistenz-induzierenden Wirksamkeit bespritzt man isolierte Blätter/Blattstücke mit der Wirkstoffzubereitung bis zur Tropfnässe. Nach einer Vorbehandlungsdauer von 3 Tagen werden die Pflanzen mit einer wäßrigen Sporensuspension von Plasmopara viticola inokuliert. Isolierte Blätter bzw. Blattstücke werden in geeigneten, belichteten Kammern bei 20 bis 22 °C und hoher Luftfeuchte (95-100 %) für 6 Tage inkubiert. Anschließend werden die Pflanzen für 1 Tag bei 100 % relativer Luftfeuchte aufgestellt.

Die Auswertung erfolgt 7 Tage nach der Inokulation.

Wirkstoffe, Wirkstoffkonzentrationen und Versuchsergebnisse gehen aus der folgenden Tabelle hervor.

Tabelle A

Plasmopara-Test an Blattstücken von Reben / protektiv (Resistenzinduktion)

| Wirkstoff | Wirkungsgrad in % der unbehandelten Kontrolle bei einer Wirkstoffkonzentration von 100 ppm |
|---|---|
| (Kontrolle) | 0 |
| Bekannt: | |
| 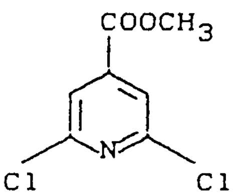 (A) | 15 |
| Erfindungsgemäß: | |
| 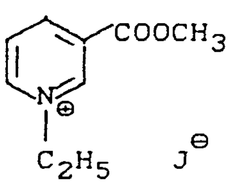 (2) | 70 |

Tabelle A (Fortsetzung)

Plasmopara-Test an Blattstücken von Reben / protektiv (Resistenzinduktion)

| Wirkstoff | Wirkungsgrad in % der unbehandelten Kontrolle bei einer Wirkstoffkonzentration von 100 ppm |
|---|---|
| (3) 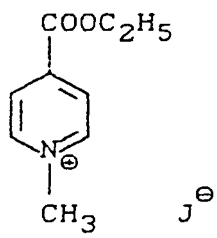 | 48 |
| (4) 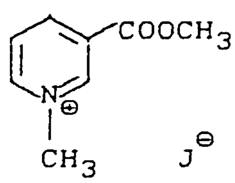 | 69 |
| (5) 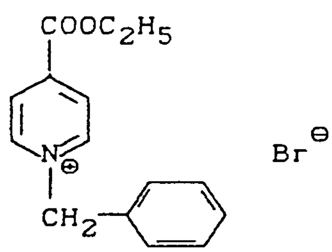 | 70 |
| (6) 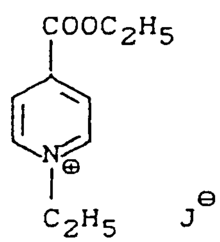 | 79 |

Beispiel B

Botrytis-Test an Blattstücken von Reben / protektiv (Resistenzinduktion)

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung der Resistenz-induzierenden Wirksamkeit bespritzt man isolierte Blätter/Blattstücke mit der Wirkstoffzubereitung bis zur Tropfnässe. Nach einer Vorbehandlungsdauer von 3 Tagen werden die Pflanzen mit einer wäßrigen Sporensuspension von Botrytis cinerea inokuliert. Die isolierten Pflanzenteile (Blätter bzw. Blattstücke) werden in geeigneten, belichteten Kammern bei 20 bis 22°C und hoher Luftfeuchte (95-100 %) inkubiert.

Am 3. Tag nach der Inokulation wird die Größe der Befallsflecken auf dem Blättern ausgewertet und der Wirkungsgrad bestimmt.

Wirkstoffe, Wirkstoffkonzentrationen und Versuchsergebnisse gehen aus der folgenden Tabelle hervor.

Tabelle B

Botrytis-Test an Blattstücken von Reben / protektiv (Resistenzinduktion)

| Wirkstoff | Wirkungsgrad in % der unbehandelten Kontrolle bei einer Wirkstoffkonzentration von 100 ppm |
|---|---|
| (Kontrolle) | 0 |
| Bekannt: | |
| 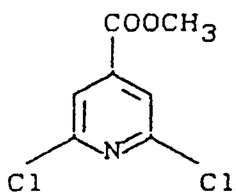 (A) | 26 |
| Erfindungsgemäß: | |
| 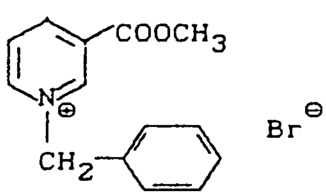 (1) | 48 |
| 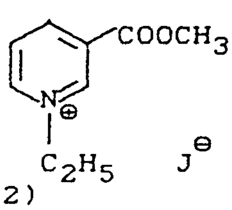 (2) | 67 |

Tabelle B (Fortsetzung)

Botrytis-Test an Blattstücken von Reben / protektiv (Resistenzinduktion)

| Wirkstoff | Wirkungsgrad in % der unbehandelten Kontrolle bei einer Wirkstoffkonzentration von 100 ppm |
|---|---|
| (3) 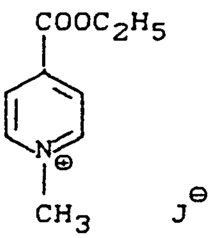 | 39 |
| (4) 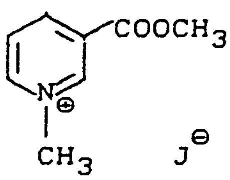 | 58 |
| (5) 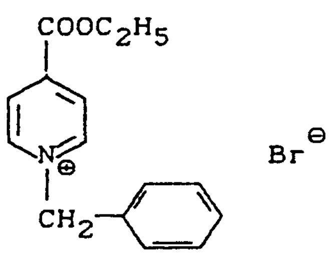 | 39 |

## Patentansprüche

1. Verwendung von Pyridinium-Salzen der Formel

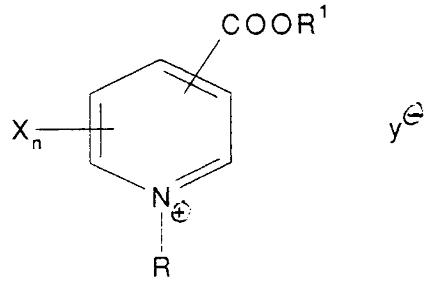

(I)

in welcher

R für Alkyl mit 1 bis 8 Kohlenstoffatomen oder für Aralkyl mit 6 bis 10 Kohlenstoffatomen im Arylteil und 1 bis 4 Kohlenstoffatomen im Alkylteil steht,

$R^1$ für Alkyl mit 1 bis 8 Kohlenstoffatomen oder für Aralkyl mit 6 bis 10 Kohlenstoffatomen im Arylteil und 1 bis 4 Kohlenstoffatomen im Alkylteil steht, wobei jeder dieser Reste im Arylteil einfach bis dreifach, gleichartig oder verschieden durch Halogen substituiert sein kann,

X für Fluor, Chlor, Brom, Iod, Alkyl mit 1 bis 8 Kohlenstoffatomen, Halogenalkyl mit 1 bis 4 Kohlenstoffatomen und 1 bis 5 gleichen oder verschiedenen Halogenatomen. Alkyoxy mit 1 bis 8 Kohlenstoffatomen, Halogenalkoxy mit 1 bis 4 Kohlenstoffatomen und 1 bis 5 gleichen oder verschiedenen Halogenatomen, Hydroxy, Mercapto, Alkylthio mit 1 bis 8 Kohlenstoffatomen, Halogenalkylthio mit 1 bis 4 Kohlenstoffatomen und 1 bis 5 gleichen oder verschiedenen Halogenatomen, Amino, Alkylamino mit 1 bis 6 Kohlenstoffatomen, Dialkylamino mit 1 bis 6 Kohlenstoffatomen in jeder Alkylgruppe, Phenylamino, Benzylamino oder gegebenenfalls durch Halogen und/oder Alkyl mit 1 bis 4 Kohlenstoffatomen substituiertes Phenyl steht,

n für 0, 1, 2, 3 oder 4 steht und

$Y^{\ominus}$ für Halogenid, Alkylsulfat mit 1 bis 4 Kohlenstoffatomen, Tetrafluoroborat, für ein Äquivalent eines Sulfatanions, für Alkylsulfonat mit 1 bis 4 Kohlenstoffatomen, gegebenenfalls durch Methyl substituiertes Phenylsulfonat, Nitrat, Alkylcarboxylat mit 1 bis 4 Kohlenstoffatomen in der Alkylgruppe oder ein Äquivalent eines Oxalatanions steht,

zur Erzeugung von Resistenz in Pflanzen gegen Befall durch Mikroorganismen.

## Claims

1. Use of pyridinium salts of the formula

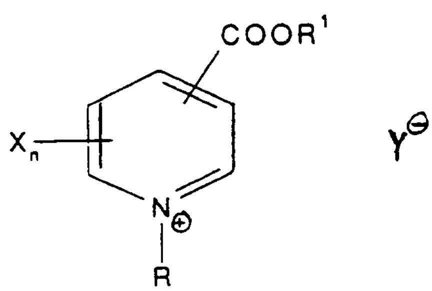

(I)

in which

R represents alkyl having 1 to 8 carbon atoms or aralkyl having 6 to 10 carbon atoms in the aryl moiety and 1 to 4 carbon atoms in the alkyl moiety,

$R^1$ represents alkyl having 1 to 8 carbon atoms or aralkyl having 6 to 10 carbon atoms in the aryl moiety and 1 to 4 carbon atoms in the alkyl moiety, it being possible for each of these radicals to be monosubstituted to trisubstituted in the aryl moiety by identical or different halogen substituents,

X represents fluorine, chlorine, bromine, iodine, alkyl having 1 to 8 carbon atoms, halogenoalkyl having 1 to 4 carbon atoms and 1 to 5 identical or different halogen atoms, alkoxy having 1 to 8 carbon atoms, halogenoalkoxy having 1 to 4 carbon atoms and 1 to 5 identical or different halogen atoms, hydroxyl, mercapto, alkylthio having 1 to 8 carbon atoms, halogenoalkylthio having 1 to 4 carbon atoms and 1 to 5 identical or different halogen atoms, amino, alkylamino having 1 to 6 carbon atoms, dialkylamino having 1 to 6 carbon atoms in each alkyl group, phenylamino, benzylamino or phenyl which is optionally substituted by halogen and/or alkyl having 1 to 4 carbon atoms,

n represents 0, 1, 2, 3 or 4 and

$Y^{\ominus}$ represents halide, alkyl sulphate having 1 to 4 carbon atoms, tetrafluoroborate, an equivalent of a sulphate anion, alkylsulphonate having 1 to 4 carbon atoms, optionally methyl-substituted phenylsulphonate, nitrate, alkylcarboxylate having 1 to 4 carbon atoms in the alkyl group, or an equivalent of an oxalate anion,

for generating resistance in plants against attack by microorganisms.

## Revendications

**1.** Utilisation de sels de pyridinium de formule

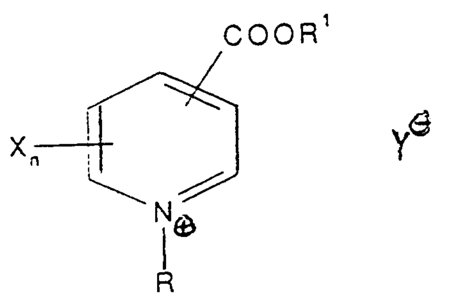

(I)

dans laquelle

R représente un groupe alkyle contenant de 1 à 8 atomes de carbone ou un groupe aralkyle contenant de 6 à 10 atomes de carbone dans la fraction aryle et de 1 à 4 atomes de carbone dans la fraction alkyle,

$R^1$ représente un groupe alkyle contenant de 1 à 8 atomes de carbone ou un groupe aralkyle contenant de 6 à 10 atomes de carbone dans la fraction aryle et de 1 à 4 atomes de carbone dans la fraction alkyle, chacun de ces radicaux dans la fraction aryle pouvant être substitué de 1 à 3 fois de manière identique ou différente par un atome d'halogène,

X représente un atome de fluor, un atome de chlore, un atome de brome, un atome d'iode, un groupe alkyle contenant de 1 à 8 atomes de carbone, un groupe halogénalkyle contenant de 1 à 4 atomes de carbone et de 1 à 5 atomes d'halogène identiques ou différents, un groupe alcoxy contenant de 1 à 8 atomes de carbone, un groupe halogénalcoxy contenant de 1 à 4 atomes de carbone et de 1 à 5 atomes d'halogène identiques ou différents, un groupe hydroxyle, un groupe mercapto, un groupe alkylthio contenant de 1 à 8 atomes de carbone, un groupe halogénalkylthio contenant de 1 à 4 atomes de carbone et de 1 à 5 atomes d'halogène identiques ou différents, un groupe amino, un groupe alkylamino contenant de 1 à 6 atomes de carbone, un groupe dialkylamino contenant de 1 à 6 atomes de carbone dans chaque groupe alkyle, un groupe phénylamino, un groupe benzylamino ou encore un groupe phényle portant éventuellement un ou plusieurs substituants identiques ou différents halogéno et/ou alkyle contenant de 1 à 4 atomes de carbone,

n représente 0, 1, 2, 3 ou 4, et

$Y^{\ominus}$ représente un halogénure, un sulfate d'alkyle contenant de 1 à 4 atomes de carbone, le tétrafluoroborate, un équivalent d'un anion sulfate, un sulfonate d'alkyle contenant de 1 à 4 atomes de carbone, un sulfonate de phényle portant éventuellement un ou plusieurs substituants méthyle identiques ou différents, un nitrate, un carboxylate d'alkyle contenant de 1 à 4 atomes de carbone dans la fraction alkyle, ou encore un équivalent d'un anion oxalate,

pour générer une résistance dans des plantes contre l'attaque déclenchée par des microorganismes.